# EUROPEAN PATENT SPECIFICATION

(11) **EP 3 525 806 B1**
(45) Date of publication and mention of the grant of the patent: **15.02.2023**
(21) Application number: 17859969.2
(22) Date of filing: 12.10.2017
(51) Int. Cl.: A61K 9/19, A61K 35/28, A61K 47/26, A61K 47/42, A61K 38/46

(54) **METHOD FOR LYOPHILISING AN EXOSOME**
VERFAHREN ZUR LYOPHILISIERUNG EINES EXOSOMS
PROCÉDÉ DE LYOPHILISATION D'EXOSOMES

(30) Priority: 12.10.2016 SG 10201608511S
(43) Date of publication of application: 21.08.2019
(73) Proprietor: Agency for Science, Technology and Research, Singapore 138632 (SG)
(72) Inventor: LIM, Sai Kiang, Singapore 138648 (SG)
(74) Representative: Boult Wade Tennant LLP
(86) International application number: PCT/SG2017/050513
(87) International publication number: WO 2018/070939

(56) References cited:
- CN-A- 101 361 989
- CN-A- 104 382 827
- CN-A- 104 488 850
- CN-A- 107 245 472
- US-A1- 2004 185 524
- US-A1- 2005 048 460
- US-A1- 2010 105 133
- JOHNSEN KASPER BENDIX ET AL: "Evaluation of electroporation-induced adverse effects on adipose-derived stem cell exosomes", CYTOTECHNOLOGY, KLUWER ACADEMIC PUBLISHERS, DORDRECHT, NL, vol. 68, no. 5, 8 February 2016 (2016-02-08), pages 2125-2138, XP036055372, ISSN: 0920-9069, DOI: 10.1007/S10616-016-9952-7 [retrieved on 2016-02-08]
- FUHRMANN G. et al.: "Stability of extracellular vesicles during lyophilization - implications for their pharmaceutical use", J Extracell Vesicles, vol. 5, no. 1, 23 February 2016 (2016-02-23), page 14, XP009514159, ISSN: 2001-3078, DOI: 10.3402/jev.v5.30924
- MADDEN T.D. et al.: "Protection of large unilamellar vesicles by trehalose during dehydration: retention of vesicle contents", Biochim Biophys Acta, vol. 817, no. 1 11 July 1985 (1985-07-11), pages 67-74, XP023361365, [retrieved on 2017-11-30]
- BOSCH S. et al.: "Impact of trehalose on isolation, storage and biological activities of extracellular vesicles released from beta-cells", J Extracell Vesicles, vol. 4, no. S1, 21 April 2015 (2015-04-21) , page 43, XP009514161, ISSN: 2001-3078, DOI: 10.3402/jev.v4.27783
- QL X. et al.: "Exosomes Secreted by Human-Induced Pluripotent Stem Cell - Derived Mesenchymal Stem Cells Repair Critical-Sized Bone Defects through Enhanced Angiogenesis and Osteogenesis in Osteoporotic Rats", Int J Biol Sci, vol. 12, no. 7 25 May 2016 (2016-05-25), pages 836-849, XP055487959, [retrieved on 2017-11-30]
- FRANK J. et al.: "Best before - Lyophilisation as novel storage alternative for extracellular vesicles", J Extracell Vesicles, vol. 6, no. S 1, 15 May 2017 (2017-05-15), pages 184-185, XP009514158, ISSN: 2001-3078, DOI: 10.1080/20013078.2017.1310414
- BOSCH S. et al.: "Trehalose prevents aggregation of exosomes and cryodamage", Sci Rep, vol. 6 8 November 2016 (2016-11-08), pages 1-11, XP055500173, [retrieved on 2017-11-30]

## Description

### FIELD

This invention relates to the fields of medicine, cell biology, molecular biology and genetics. This invention relates to the field of medicine.

### BACKGROUND

Mesenchymal stem cells (MSCs) were first isolated and described as multipotent stem cells with the capacity to differentiate into at least three cell types, namely adipocytes, chondrocytes and osteocytes ³. However, they were increasingly seen as stromal support progenitor cells that differentiate into stromal support cells and secrete factors to support the stroma or other cells ^{4,5}.

Consistent with their role as stromal support cells, they are found ubiquitously in many tissues. Despite their presence in different tissue sources, MSCs share a similar characteristic phenotype ⁶ albeit with some additional features that reflect their tissue of origin ⁷. The best characterized stromal support function of MSCs is in the maintenance of hematopoietic stem cell homeostasis where MSCs help to maintain a microenvironmental niche that equilibrate the quiescence of hematopoietic stem cells, their self-renewal and differentiation with the needs of the body ⁸. MSCs are also notorious for their well-documented critical role in constructing the tumor microenvironment that in turn, is important in tumor growth and dissemination (reviewed ⁹).

Presently, MSC is a prominent cell type because of their reported regenerative potency, and its use in clinical trials has increased exponentially in recent years ¹⁰. Over the years with increasing observations from clinical and animal studies, the rationale for the clinical testing of MSCs had transition from a differentiation-based rationale to a secretion based rationale. In contrast to the differentiation-based rationale where the transplanted MSC home and engraft into the target tissue where they differentiate into functional cell type to replace injured cells, the secretion-based rationale proposes that MSC secrete factors to reduce cellular injury and enhance repair ¹¹.

The paracrine effect of MSCs was first described almost two decades ago when Haynesworth et al ¹² reported that MSCs synthesize and secrete a broad spectrum of growth factors, chemokines and cytokines that could exert significant effects on cells in their vicinity. The initial studies in MSC secretion focused mainly on small molecules such as growth factors, chemokines and cytokines as the active agents ¹¹. However, none of these promising candidate molecules fail to account for the efficacy of MSCs (reviewed ¹³). Subsequently, Bruno et al in 2009 demonstrated that MSCs secrete 80 nm to 1 µm microvesicles that protect against acute tubular injury ¹⁴ while Lai et al in 2010 showed that MSC exosomes of 40-100 nm protect against myocardial reperfusion injury. Since then, EVs have been increasingly reported as the therapeutic agent in MSC secretion ¹⁵.

Exosomes are secreted bi-lipid membrane vesicles with a diameter of 40-100 nm, a flotation density of 1.10 - 1.18 g/ml in a sucrose gradient, and have exosome-associated markers such as Alix, Tsg101, the tetraspanins, CD9, CD63 or CD81 ^{16,17}. They are the only class of extracellular known to be derived from endosomes through the invagination of the endosomal membrane to form a multivesicular body (MVB) with numerous intraluminal vesicles. The intraluminal vesicles are released as exosomes upon fusion with the plasma membrane ¹⁸.

Exosomes for therapeutic applications are commonly stored frozen at -20°C or -80°C. The shelf life of exosomes stored frozen at -20°C or -80°C is at most one year. This limits the accessibility of exosome-based therapeutics.

Methods for lyophilising exosomes are known in the art. Such methods however require storage of lyophilised lysosomes at cold temperatures, in order to preserve activity.

Exosomes may be lyophilised in phosphate buffered saline with sucrose (PBS + sucrose), as for example described by HansaBioMed (2014 catalogue). Exosomes lyophilised by such a method are said to be able to be stored at 4°C for up to a year without losing proteins or affecting particle size. However, the effect of lyophilization on protein activity or the therapeutic potency of the exosome was not addressed. It is well established that the conservation of protein structure during lyophilization does not always translate into conservation of protein activity²⁰. Accordingly, the HansaBioMed exosomes are intended to be used primarily as reference controls for FACS or western blots, and there is no disclosure in this document that the exosomes retain any biological activity such as therapeutic activity.

US 2016/0158291 A1 describes a protocol to lyophilize exosomes from cardiosphere-derived cells. The exosomes were dissolved in plasmalyte A and lyophilized in a defined freezing and drying process. The lyophilized exosomes were stored for at least 30 days at -80°C. Upon reconstitution with water, the reconstituted exosomes showed bioactivity.

CN 104382827 A describes uses of human amniotic membrane mesenchymal stem cell exosomes.

CN 104488850 A describes a method for preparing a freeze-dried powder of human amniotic membrane mesenchymal stem cell exosomes. To this date, however, there is no process to lyophilize exosomes such that their structure and therapeutic potency are preserved upon prolonged storage at ambient temperature.

There is a need for a protocol to preserve exosomes that will not only preserve their structural and biochemical integrity but also the therapeutic efficacy of exosomes for long term storage at ambient temperature.

### SUMMARY

According to a 1^{st} aspect of the present invention, we provide a method for lyophilising an exosome as set out in the claims.

The practice of this invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Femandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### BRIEF DESCRIPTION OF THE FIGURES

Figure 1 is a drawings showing the stability of CD73 enzymatic activity in lyophilized exosome
Figure 2 (comprising Figure 2A, Figure 2B and Figure 2C) is a drawings showing the effect of lyophilisation on exosome number and size.
Figure 3 is a drawings showing the cardioprotective potency of lyophilized exosomes.
Figure 4 is a drawings showing the polarization of Tregs.
Figure 5 is a drawings showing the freeze drying of exosomes on biomaterial scaffold and gamma irradiation.

### DETAILED DESCRIPTION

The invention is defined in the claims and provides a method for lyophilising an exosome, the method comprising the steps of:
(a) providing an exosome suspension in a lyophilisation buffer comprising trehalose at a concentration of 4% w/v ;
(b) freezing the exosome suspension; and
(c) removing water from the frozen suspension by freeze drying;
in which the lyophilised exosome exhibits at least one biological activity of an exosome.

The lyophilisation buffer comprises trehalose at a concentration of 4% w/v. The lyophilisation buffer may include compounds such as sugars or polyols, polymers, surfactants, buffers, amino acids, chelating complexes and inorganic salts. Compounds such as acetone or poloxamer 188 may also be included. The compounds may perform a function as an excipient, for example.

The lyophilisation buffer may include one or more of these compounds, for example a combination of lactose and glucose, at, for example, a specified mass ratio such as 2:1 glucose:lactose.

The method is such that the lyophilised exosome exhibits at least one biological activity of an exosome. The biological activity may comprise a biochemical activity such as CD73 enzymatic activity. The biological activity may comprise a therapeutic activity, such as cardioprotective activity.

The exosome may exhibit 5% or more of the biological activity when reconstituted. The exosome may exhibit 10% or more of the biological activity when reconstituted. The exosome may exhibit 15% or more of the biological activity when reconstituted. The exosome may exhibit 20% or more of the biological activity when reconstituted. The exosome may exhibit 25% or more of the biological activity when reconstituted. The exosome may exhibit 30% or more of the biological activity when reconstituted. The exosome may exhibit 35% or more of the biological activity when reconstituted. The exosome may exhibit 40% or more of the biological activity when reconstituted. The exosome may exhibit 45% or more of the biological activity when reconstituted. The exosome may exhibit 50% or more of the biological activity when reconstituted. The exosome may exhibit 55% or more of the biological activity when reconstituted. The exosome may exhibit 60% or more of the biological activity when reconstituted. The exosome may exhibit 65% or more of the biological activity when reconstituted. The exosome may exhibit 70% or more of the biological activity when reconstituted. The exosome may exhibit 75% or more of the biological activity when reconstituted. The exosome may exhibit 80% or more of the biological activity when reconstituted. The exosome may exhibit 85% or more of the biological activity when reconstituted. The exosome may exhibit 90% or more of the biological activity when reconstituted. The exosome may exhibit or 95% or more of the biological activity when reconstituted.

The exosome may exhibit the biological activity when stored at a temperature of 0°C or higher. The exosome may exhibit the biological activity when stored at a temperature of 4°C or higher. The exosome may exhibit the biological activity when stored at a temperature of 10°C or higher. The exosome may exhibit the biological activity when stored at a temperature of 15°C or higher. The exosome may exhibit the biological activity when stored at a temperature of 20°C or higher. The exosome may exhibit the biological activity when stored at a temperature of 25°C or higher. The exosome may exhibit the biological activity when stored at a temperature of 30°C or higher. The exosome may exhibit the biological activity when stored at a temperature of 35°C or higher. The exosome may exhibit the biological activity when stored at a temperature of 40°C or higher.

The exosome may exhibit the biological activity when stored for 1 day or more. The exosome may exhibit the biological activity when stored for 5 days or more. The exosome may exhibit the biological activity when stored for 7 days or more. The exosome may exhibit the biological activity when stored for 10 days or more. The exosome may exhibit the biological activity when stored for 14 days or more. The exosome may exhibit the biological activity when stored for 20 days or more. The exosome may exhibit the biological activity when stored for 21 days or more. The exosome may exhibit the biological activity when stored for 25 days or more. The exosome may exhibit the biological activity when stored for 28 days or more. The exosome may exhibit the biological activity when stored for 30 days or more. The exosome may exhibit the biological activity when stored for 35 days or more. The exosome may exhibit the biological activity when stored for or 40 days or more.

The biological activity may comprise CD73 enzymatic activity.

The exosome may exhibit 20% or more CD73 enzymatic activity. The exosome may exhibit 25% or more CD73 enzymatic activity. The exosome may exhibit 30% or more CD73 enzymatic activity. The exosome may exhibit 35% or more CD73 enzymatic activity. The exosome may exhibit 40% or more CD73 enzymatic activity. The exosome may exhibit 45% or more CD73 enzymatic activity. The exosome may exhibit 50% or more CD73 enzymatic activity. The exosome may exhibit 55% or more CD73 enzymatic activity. The exosome may exhibit 60% or more CD73 enzymatic activity. The exosome may exhibit that CD73 enzymatic activity after 55 days of storage at 40°C. The exosome may exhibit that CD73 enzymatic activity as compared to the activity after 1 day of storage at 40°C.

The biological activity may comprise induction of polarization of CD4+ T cells to regulatory T cells (Tregs).

The exosome may exhibit 20% or more activity to induce polarization of CD4+ T cells to regulatory T cells (Tregs). The exosome may exhibit 25% or more activity to induce polarization of CD4+ T cells to regulatory T cells (Tregs). The exosome may exhibit 30% or more activity to induce polarization of CD4+ T cells to regulatory T cells (Tregs). The exosome may exhibit 35% or more activity to induce polarization of CD4+ T cells to regulatory T cells (Tregs). The exosome may exhibit 40% or more activity to induce polarization of CD4+ T cells to regulatory T cells (Tregs). The exosome may exhibit 45% or more activity to induce polarization of CD4+ T cells to regulatory T cells (Tregs). The exosome may exhibit 50% or more activity to induce polarization of CD4+ T cells to regulatory T cells (Tregs). The exosome may exhibit 55% or more activity to induce polarization of CD4+ T cells to regulatory T cells (Tregs). The exosome may exhibit 60% or more activity to induce polarization of CD4+ T cells to regulatory T cells (Tregs). The exosome may exhibit that activity to induce polarization of CD4+ T cells to regulatory T cells (Tregs) after 3 weeks of storage at 40°C. The exosome may exhibit that activity to induce polarization of CD4+ T cells to regulatory T cells (Tregs) when compared with a non-lyophilized exosome stored at -80°C.

The biological activity may comprise cardioprotection. The cardioprotection may be assayed using an *ex vivo* Landerdorf model of ischemia-reperfusion injury. The cardioprotection may be assayed by the degree of hypercontracture over post-reperfusion time.

The exosome may exhibit 20% or more cardioprotective activity. The exosome may exhibit 25% or more cardioprotective activity. The exosome may exhibit 30% or more cardioprotective activity. The exosome may exhibit 35% or more cardioprotective activity. The exosome may exhibit 40% or more cardioprotective activity. The exosome may exhibit 45% or more cardioprotective activity. The exosome may exhibit 50% or more cardioprotective activity. The exosome may exhibit 55% or more cardioprotective activity. The exosome may exhibit 60% or more cardioprotective activity. The exosome may exhibit that cardioprotective activity when compared with non-lyophilized exosome stored at -80°C.

Step (b) may comprise freezing the exosome suspension at a temperature of -10°C or lower. Step (b) may comprise freezing the exosome suspension at a temperature of -15°C or lower. Step (b) may comprise freezing the exosome suspension at a temperature of -20°C or lower.

Step (b) may comprise freezing the exosome suspension for 1 hour or more. Step (b) may comprise freezing the exosome suspension for 2 hours or more. Step (b) may comprise freezing the exosome suspension for 3 hours or more.

Step (c) may comprise freeze drying at a temperature of -10°C or lower. Step (c) may comprise freeze drying at a temperature of -15°C or lower. Step (c) may comprise freeze drying at a temperature of -20°C or lower. Step (c) may comprise freeze drying at a temperature of -25°C or lower. Step (c) may comprise freeze drying at a temperature of -30°C or lower. Step (c) may comprise freeze drying at a temperature of -35°C or lower. Step (c) may comprise freeze drying at a temperature of -40°C or lower. Step (c) may comprise freeze drying at a temperature of -45°C or lower.

Step (c) may comprise freeze drying at a pressure of 1 mbar or lower. Step (c) may comprise freeze drying at a pressure of 0.5 mbar or lower. Step (c) may comprise freeze drying at a pressure of 0.25 mbar or lower. Step (c) may comprise freeze drying at a pressure of 0.1 mbar or lower. Step (c) may comprise freeze drying at a pressure of 0.05 mbar or lower.

Step (c) may comprise freeze drying at a temperature of -10°C or lower. Step (c) may comprise freeze drying at a temperature of -15°C or lower. Step (c) may comprise freeze drying at a temperature of -20°C or lower. Step (c) may comprise freeze drying at a temperature of -25°C or lower. Step (c) may comprise freeze drying at a temperature of -30°C or lower. Step (c) may comprise freeze drying at a temperature of -35°C or lower. Step (c) may comprise freeze drying at a temperature of -40°C or lower. Step (c) may comprise freeze drying at a temperature of -45°C or lower.

Step (c) may comprise freeze drying for 5 hours or longer. Step (c) may comprise freeze drying for 10 hours or longer. Step (c) may comprise freeze drying for 15 hours or longer. Step (c) may comprise freeze drying for 20 hours or longer.

The exosome may comprise a mesenchymal stem cell (MSC) exosome.

Step (b) may comprise freezing the exosome suspension at about -20°C for about 3 hours. Step (c) may comprise freeze drying at < 0.05mbar for about 12 to 24 hours

The method may further comprise a step before step (c) of transferring the frozen suspension to a freeze dryer pre-cooled to -48°C.

The method may further comprise storing the lyophilised exosome at a temperature of 4°C or higher. The method may further comprise storing the lyophilised exosome at a temperature of 10°C or higher. The method may further comprise storing the lyophilised exosome at a temperature of 15°C or higher, 20°C or higher. The method may further comprise storing the lyophilised exosome at a temperature of 25°C or higher, 30°C or higher. The method may further comprise storing the lyophilised exosome at a temperature of 35°C or higher or 40°C or higher.

The method may further comprise storing the lyophilised exosome in substantially dry conditions, such as in a desiccator.

The substantially dry conditions may comprise storage in air with a relative humidity of 5% or less. The substantially dry conditions may comprise storage in air with a relative humidity of 4% or less. The substantially dry conditions may comprise storage in air with a relative humidity of 3% or less. The substantially dry conditions may comprise storage in air with a relative humidity of 2% or less. The substantially dry conditions may comprise storage in air with a relative humidity of 1% or less.

The method may further comprise storing the lyophilised exosome in an inert gas. The inert gas may comprise nitrogen.

The lyophilisation buffer may further comprise a phosphate buffer. The phosphate buffer may be a pH 6.5-7.5 buffer. The lyophilisation buffer may further comprise an amino acid. The amino acid may comprise L-glutamic acid. The L-glutamic acid may be at a concentration of 0.0049M. The lyophilisation buffer may further comprise a protein binding stabiliser. The protein binding stabiliser may comprise human serum albumin. The human serum albumin may be at a concentration of 1% w/v.

The method may further comprise a step of viral clearance. The viral clearance may be by exposure to gamma radiation. The viral clearance may be by exposure to X-ray irradiation. The viral clearance may be by exposure to ethylene oxide.

The method may comprise a step before step (b) of coating an object with the exosome suspension. The method may be such that removal of water from the frozen suspension deposits the exosome on the object.

The object may comprise a scaffold. The object may be made of a biocompatible material.

The object comprise a collagen membrane. The object comprise a surgical dressing. The object comprise a prosthetic bone replacement device. The object comprise a cardiovascular device. The object comprise a stent and a balloon catheter.

### LYOPHILISATION

Freeze-drying, also known as lyophilisation, lyophilization, or cryodesiccation, is a dehydration process typically used to preserve a perishable material or make the material more convenient for transport.

Freeze-drying works by freezing the material and then reducing the surrounding pressure to allow the frozen water in the material to sublimate directly from the solid phase to the gas phase.

Lyophilisation is therefore a stabilising process in which the formulation is first frozen, i.e., a separation of the solvent and the solutes, and then the concentration of the solvent, mainly water, is reduced first by sublimation (primary drying) and then by desorption (secondary drying) to levels that will no longer support biological growth or chemical reaction.

Lyophilisation is also known as free drying. The lyophilisation process may comprise up to four stages: pretreatment, freezing, primary drying, and secondary drying.

### Pretreatment

Pretreatment includes any method of treating the product prior to freezing.

This may include concentrating the product, formulation revision (i.e., addition of components to increase stability, preserve appearance, and/or improve processing), decreasing a high-vapor-pressure solvent, or increasing the surface area.

### Freezing

Freezing may be conducted by placing the material in a freeze-drying flask and rotating the flask in a bath, called a shell freezer, which is cooled by mechanical refrigeration, dry ice in aqueous methanol, or liquid nitrogen.

On a larger scale, freezing may be conducted using a freeze-drying machine. In this step, it is important to cool the material below its triple point, the lowest temperature at which the solid, liquid and gas phases of the material can coexist. This ensures that sublimation rather than melting will occur in the following steps. Larger crystals are easier to freeze-dry. To produce larger crystals, the product should be frozen slowly or can be cycled up and down in temperature. This cycling process is called annealing. However, in the case of food, or objects with formerly-living cells, large ice crystals will break the cell walls, resulting in the destruction of more cells, which can result in increasingly poor texture and nutritive content.

In this case, the freezing may be conducted rapidly, in order to lower the material to below its eutectic point quickly, thus avoiding the formation of ice crystals. Usually, the freezing temperatures are between -50 °C and -80 °C (-58 °F and -112 °F) . The freezing phase is usually the most critical in the whole freeze-drying process.

Amorphous materials do not have a eutectic point, but they do have a critical point, below which the product must be maintained to prevent melt-back or collapse during primary and secondary drying.

The exosome suspension may be frozen at any suitable temperature, such as a temperature below 0°C, such as at -5°C or lower, -10°C or lower, -15°C or lower or -20°C or lower, -30°C or lower, -35°C or lower or -40°C or lower, -45°C or lower or -50°C or lower.

The freezing may take place over any suitable time period. For example, the exosome suspension may be frozen for 30 minutes or more, 1 hour or more, 1.5 hours or more, 2 hours or more, 2.5 hours or more, 3 hours or more, 3.5 hours or more or 4 hours or more.

### Primary Drying

During the primary drying phase, the pressure is lowered (to the range of a few millibars), and enough heat is supplied to the material for the ice to sublime.

The amount of heat necessary can be calculated using the sublimating molecules' latent heat of sublimation. In this initial drying phase, about 95% of the water in the material is sublimated. This phase may be slow (up to several days), as, if too much heat is added, the material's structure could be altered.

In this phase, pressure is controlled through the application of partial vacuum. The vacuum speeds up the sublimation, making it useful as a deliberate drying process. Furthermore, a cold condenser chamber and/or condenser plates provide a surface(s) for the water vapour to resolidify on. This condenser plays no role in keeping the material frozen; rather, it prevents water vapor from reaching the vacuum pump, which could degrade the pump's performance. Condenser temperatures are typically below -50 °C (-58 °F).

### Secondary Drying

A secondary drying phase may also be included. This phase aims to remove unfrozen water molecules, since the ice was removed in the primary drying phase. This part of the freeze-drying process is governed by the material's adsorption isotherms.

In this phase, the temperature is raised higher than in the primary drying phase, and can even be above 0 °C, to break any physico-chemical interactions that have formed between the water molecules and the frozen material. Usually the pressure is also lowered in this stage to encourage desorption (typically in the range of microbars, or fractions of a pascal). However, there are products that benefit from increased pressure as well.

After the freeze-drying process is complete, the vacuum is usually broken with an inert gas, such as nitrogen, before the material is sealed.

At the end of the operation, the final residual water content in the product is extremely low, around 1% to 4%.

The one or more drying phases may take place at any suitable temperature, such as a temperature of -10°C or lower, -15°C or lower, -20°C or lower, -25°C or lower, -30°C or lower, - 35°C or lower, -40°C or lower or -45°C or lower. The pressure at which the drying phase is conducted may be varied, but is typically below atmospheric pressure. For example, the one or more drying phases may be conducted at a pressure of 1 mbar or lower, 0.5 mbar or lower, 0.25 mbar or lower, 0.1 mbar or lower or 0.05 mbar or lower. The one or more drying phases may take any suitable amounts of time. For example, the exosome suspension may be dried for 1 hour or more, 2 hours or more, 3 hours or more, 4 hours or more, 5 hours or more, 6 hours or more, 7 hours or more, 8 hours or more, 9 hours or more, 10 hours or more, 11 hours or more, 12 hours or more, 13 hours or more, 14 hours or more, 15 hours or more, 16 hours or more, 17 hours or more, 18 hours or more, 19 hours or more or 20 hours or more.

### Viral Clearance

The method of producing a lyophilised exosome may optionally include a step of removing contaminants such as microorganisms. The decontamination step may comprise viral clearance, to remove viruses present with the lyophilised exosome.

The decontamination step may take place before or after any of the steps set out above.

The exosomes may be exposed to gamma radiation, X-ray irradiation or ethylene oxide in order to perform viral clearance.

### Deposition

The method of producing a lyophilised exosome may optionally include a step of depositing the exosome on an object. Thus, a scaffold is introduced in the environment of the exosome, so that it is deposited on the object as it is dried.

Suitably, the deposition step takes place before the step of freezing the exosome suspension. An object on which it is desired that the exosomes are deposited is added to the exosome suspension in the lyophilisation buffer and the suspension is frozen around the object. In this way, the object receives a layer of exosome as the freezing and drying takes place, and as the water is removed. Removal of water deposits the exosome on the object.

The object may comprise a suitable scaffold, such as a biocompatible scaffold. Biocompatible scaffolds are known to the person skilled in the art, and are described for example in O'Brien (2011). Biomaterials & scaffolds for tissue engineering. Materials Today, 14, 3, pp 88-95.

Examples of biocompatible materials which may be suitably used in the methods described in this document include collagen membranes, surgical dressings, prosthetic bone replacement devices, cardiovascular devices, stents and balloon catheters.

### LYOPHILISATION BUFFER

For the purposes of lyophilisation, an exosome is suspended in a lyophilisation buffer.

The lyophilisation buffer may comprise a buffer, for example to stabilise pH. The buffer may comprise a phosphate buffer, such as phosphate buffered saline (PBS). The buffer may maintain physiological pH at for example pH 6.5-7.5.

The buffer may comprise an amino acid for example L-glutamic acid, at a suitable concentration such as at 0.0049M. The buffer may comprise a protein binding stabiliser, for example human serum albumin such as at 1% w/v.

### SUGAR

The lyophilisation buffer comprises trehalose at a concentration of 4% w/v and may further comprise a sugar. The sugar may comprise a monosaccharide such as fructose, glucose or galactose, or a di-saccharide, as described in more detail below.

The concentration of the sugar may be 10% w/v or less, such as 9% w/v or less, 8.5% w/v or less, 8% w/v or less, 7.5% w/v or less, 7% w/v or less, 6.5% w/v or less, 6% w/v or less, 5.5% w/v or less, 5% w/v or less, 4.5% w/v or less, 4% w/v or less, 3.5% w/v or less, 3% w/v or less, 2.5% w/v or less, 2% w/v or less, 1.5% w/v or less or 1% w/v or less.

The sugar may be present in the lyophilisation buffer at a concentration of for example 4% w/v.

### DI-SACCHARIDE

The sugar in the lyophilisation buffer comprises the di-saccharide trehalose.

A disaccharide (also called a double sugar or biose) is the sugar formed when two monosaccharides (simple sugars) are joined by glycosidic linkage.

Reducing disaccharides, in which one monosaccharide, the reducing sugar of the pair, still has a free hemiacetal unit that can perform as a reducing aldehyde group; cellobiose and maltose are examples of reducing disaccharides, each with one hemiacetal unit, the other occupied by the glycosidic bond, which prevents it from acting as a reducing agent.

Non-reducing disaccharides, in which the component monosaccharides bond through an acetal linkage between their anomeric centers. This results in neither monosaccharide being left with a hemiacetal unit that is free to act as a reducing agent. Sucrose and trehalose are examples of non-reducing disaccharides because their glycosidic bond is between their respective hemiacetal carbon atoms. The reduced chemical reactivity of the non-reducing sugars in comparison to reducing sugars, may be of advantage where stability in storage is important.

Di-saccharides may comprise for example sucrose (also known as table sugar, cane sugar, beet sugar, or saccharose), lactulose, lactose (milk sugar), maltose (malt sugar), trehalose, cellobiose, chitobiose, kojibiose, nigerose, isomaltose, β,β-trehalose, α,β-trehalose, sophorose, laminaribiose, gentiobiose, turanose, maltulose, palatinose, gentiobiulose, mannobiose, melibiose, melibiulose, rutinose, rutinulose or xylobiose.

The lyophilisation buffer comprises trehalose at a concentration of 4% w/v.

### TREHALOSE

The lyophilisation buffer comprises trehalose at a concentration of 4% w/v.

Trehalose, also known as mycose or tremalose, is a natural alpha-linked disaccharide formed by an α,α-1,1-glucoside bond between two α-glucose units.

Trehalose may be derived from corn starch. There are at least 3 biological pathways for trehalose biosynthesis.

Trehalose is a nonreducing sugar formed from two glucose units joined by a 1-1 alpha bond, giving it the name of α-D-glucopyranosyl-(1→1)-α-D-glucopyranoside. The bonding makes trehalose very resistant to acid hydrolysis, and therefore is stable in solution at high temperatures, even under acidic conditions.

The bonding also keeps nonreducing sugars in closed-ring form, such that the aldehyde or ketone end groups do not bind to the lysine or arginine residues of proteins (a process called glycation).

Trehalose is less soluble than sucrose, except at high temperatures (>80 °C). Trehalose forms a rhomboid crystal as the dihydrate, and has 90% of the calorific content of sucrose in that form. Anhydrous forms of trehalose readily regain moisture to form the dihydrate. Anhydrous forms of trehalose can show interesting physical properties when heat-treated.

Trehalose aqueous solutions show a concentration-dependent clustering tendency. Owing to their ability to form hydrogen bonds between one another, they self-associate in water to form clusters of various sizes. All-atom molecular dynamics simulations have shown that when reaching a concentration of 1.5-2.2 molar, the trehalose molecular clusters percolate and form large, continuous aggregates within the system.

Trehalose directly interacts with nucleic acids, facilitates melting of double stranded DNA and stabilizes single-stranded nucleic acids.

### STORAGE

The lyophilized exosome may be stored at any suitable temperature, for example, below freezing. The lyophilised exosome may be stored at for example -80C, -70C, -50C, -40C, -30C, - 20C or -10C.

The lyophilised exosome may be stored in a frost-free freezer without appreciable temperature fluctuation for this purpose.

The lyophilised exosome may be stored at or above freezing conditions, such as 0C, 1C, 2C, 3C, 4C, 5C, 6C, 7C, 8C, 9C, 10C, 11C, 12C, 13C, 14C, 15C, 16C, 17C, 81C, 19C, 10C, 21C, 22C, 23C, 24C, 25C, 26C, 27C, 28C, 29C, 30C, 31C, 32C, 33C, 34C, 35C, 36C, 37C, 38C, 39C or 50C.

The lyophilised exosome may be stored at standard pressure, at atmospheric pressure or at elevated or decreased pressure. The lyophilised exosome may be stored under an inert gas, such as a noble gas or nitrogen.

The relative humidity of the storage conditions may be carefully monitored and controlled. For example, the lyophilised exosome may be stored in relatively dry conditions, with low relative humidity, such as 19% or below, 18% or below, 17% or below, 16% or below, 15% or below, 14% or below, 13% or below, 12% or below, 11% or below, 10% or below, 9% or below, 8% or below, 7% or below, 6% or below, 5% or below, 4% or below, 3% or below, 2% or below or 1% or below relative humidity.

The relative humidity of the storage conditions may be adjusted by means known in the art. Chemical or physical means may be used to remove moisture from the atmosphere surrounding the lyophilised exosome. For example, the lyophilised exosome may be stored in a desiccator, for example over a desiccant such as calcium chloride.

The lyophilised exosome may be stored for extended periods, such as 1 day or more, 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more, 16 days or more, 17 days or more, 18 days or more, 19 days or more, 20 days or more, 21 days or more, 22 days or more, 23 days or more, 24 days or more, 25 days or more, 26 days or more, 27 days or more, 28 days or more, 29 days or more, 30 days or more or 31 days or more.

The lyophilised exosome may be stored for 1 week or more, 2 weeks or more, 3 weeks or more, 4 weeks or more, 5 weeks or more, 6 weeks or more, 7 weeks or more, 8 weeks or more, 9 weeks or more, 10 weeks or more, 11 weeks or more, 12 weeks or more, 13 weeks or more, 14 weeks or more, 15 weeks or more, 16 weeks or more, 17 weeks or more, 18 weeks or more, 19 weeks or more, 20 weeks or more, 21 weeks or more, 22 weeks or more, 23 weeks or more, 24 weeks or more, 25 weeks or more, 26 weeks or more, 27 weeks or more, 28 weeks or more, 29 weeks or more, 30 weeks or more, 31 weeks or more, 32 weeks or more, 33 weeks or more, 34 weeks or more, 35 weeks or more, 36 weeks or more, 37 weeks or more, 38 weeks or more, 39 weeks or more, 40 weeks or more, 41 weeks or more, 42 weeks or more, 43 weeks or more, 44 weeks or more, 45 weeks or more, 46 weeks or more, 47 weeks or more, 48 weeks or more, 49 weeks or more, 50 weeks or more, 51 weeks or more or 52 weeks or more.

The lyophilised exosome may be stored for 1 month or more, 2 months or more, 3 months or more, 4 months or more, 5 months or more, 6 months or more, 7 months or more, 8 months or more, 9 months or more, 10 months or more, 11 months or more or 12 months or more.

The lyophilised exosome may be stored for 1 year or more, 2 years or more, 3 years or more, 4 years or more, 5 years or more, 6 years or more, 7 years or more, 8 years or more, 9 years or more, 10 years or more, 11 years or more, 12 years or more, 13 years or more, 14 years or more, 15 years or more, 16 years or more, 17 years or more, 18 years or more, 19 years or more, 20 years or more, 21 years or more, 22 years or more, 23 years or more, 24 years or more, 25 years or more, 26 years or more, 27 years or more, 28 years or more, 29 years or more or 30 years or more.

### RE-CONSTITUTION

The lyophilised exosome may be re-constituted after storage. The re-constitution process may comprise re-addition of a medium such as water or a buffer to the lyophilised product.

The medium may be added gradually for example. The activity of the re-constituted exosome may be monitored throughout the process.

The medium may comprise distilled water or a buffer such as phosphate buffered saline (PBS).

If the lyophilised exosome was stored in low temperature conditions, the lyophilised exosome may be allowed to warm up to the desired temperature, such as room temperature. The warming up process may take place concurrently or sequentially with the addition of medium.

The lyophilised exosome may be reconstituted by the addition of medium such as dH2O or 1 × PBS. After the addition of dH2O or PBS, the vial may be recapped and briefly vortexed to ensure that that the liquid has been evenly distributed and has covered the entire inside of the vial so all lyophilized exosome goes into solution.

After vortexing, the solution may be centrifuged again to pellet the solution.

### RETENTION OF ACTIVITY

The lyophilised exosome may retain all, or substantially all, or a significant percentage of its activity, after storage at the above times and conditions. The retained activity may comprise a biological activity of the exosome, as described elsewhere in this document.

The lyophilised exosome may retain 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more or 99% or more of its activity on re-constitution.

The activity of the reconstituted exosome may be compared to the activity of the exosome prior to lyophilisation, as described above. Alternatively, or in addition, the activity of the reconstituted exosome may be compared to the activity of an exosome which has not been subject to the lyophilisation process, but stored at -80°C for the same amount of time. We refer to this as a "non-lyophilized exosome".

The biological activity may comprise an enzymatic activity, such as CD73 activity, or a physiological or therapeutic activity, such as cardioprotection.

Cardioprotection may be assayed using an *ex vivo* Landerdorf model of ischemia-reperfusion injury, preferably by the degree of hypercontracture over post-reperfusion time.

### EXOSOMES

The exosome for lyophilication may be derived from a number of different sources. Exosomes from different sources may be suitable for lyophilisation according to the methods disclosed in this document.

The exosome may for example be derivable from a stem cell such as a mesenchymal stem cell (MSC), as described below and in WO 2009/105044.

Exosomes are small membrane vesicles formed in late endocytic compartments (multivesicular bodies) first described to be secreted by reticulocytes in 1983 and subsequently found to be secreted by many cells types including various haematopoietic cells, tumours of haematopoietic or non-haematopoietic origin and epithelial cells. They are distinct entities from the more recently described `ribonuclease complex' also named exosome.

Exosomes may be defined by a number of morphological and biochemical parameters. Accordingly, the exosome described here may comprise one or more of these morphological or biochemical parameters.

Exosomes are classically defined as "saucer-like" vesicles or a flattened sphere limited by a lipid bilayer with diameters of 40-100 nm and are formed by inward budding of the endosomal membrane. Like all lipid vesicles and unlike protein aggregates or nucleosomal fragments that are released by apoptotic cells, exosomes have a density of -1.13 - 1.19 g/ml and float on sucrose gradients. Exosomes are enriched in cholesterol and sphingomyelin, and lipid raft markers such as GM1, GM3, flotillin and the src protein kinase Lyn suggesting that their membranes are enriched in lipid rafts.

The molecular composition of exosomes from different cell types and of different species has been examined. In general, exosomes contain ubiquitous proteins that appear to be common to all exosomes and proteins that are cell-type specific. Also, proteins in exosomes from the same cell-type but of different species are highly conserved. The ubiquitous exosome-associated proteins include cytosolic proteins found in cytoskeleton e.g. tubulin, actin and actin-binding proteins, intracellular membrane fusions and transport e.g. annexins and rab proteins, signal transduction proteins e.g. protein kinases, 14-3-3 and heterotrimeric G proteins, metabolic enzymes e.g. peroxidases, pyruvate and lipid kinases, and enolase-1 and the family of tetraspanins e.g. CD9, CD63, CD81 and CD82. The tetraspannins are highly enriched in exosomes and are known to be involved in the organization of large molecular complexes and membrane subdomains.

Examples of cell-type specific proteins in exosomes are MHC class II molecules in exosomes from MHC class II-expressing cells, CD86 in dendritic cell-derived exosomes, T-cell receptors on T-cell-derived exosomes etc. Notably, exosomes do not contain proteins of nuclear, mitochondrial, endoplasmic-reticulum or Golgi-apparatus origin. Also, highly abundant plasma membrane proteins are absent in exosomes suggesting that they are not simply fragments of the plasma membrane. Many of the reported ubiquitous exosome-associated proteins are also present in the proteomic profile of the hESC-MSC secretion.

Exosomes are also known to contain mRNA and microRNA, which can be delivered to another cell, and can be functional in this new location. The physiological functions of exosome remain poorly defined. It is thought to help eradicate obsolete proteins, recycle proteins, mediate tramission of infectious particles such as prions and viruses, induce complement resistance, facilitate immune cell-cell communication and transmit cell signaling. Exosomes have been used in immunotherapy for treatment of cancer.

### Exosome Molecular Weight

The exosome may have a molecular weight of greater than 100 kDa. It may have a molecular weight of greater than 500 kDa. For example, it may have a molecular weight of greater than 1000 kDa.

The molecular weight may be determined by various means. In principle, the molecular weight may be determined by size fractionation and filtration through a membrane with the relevant molecular weight cut-off. The exosome size may then be determined by tracking segregation of component proteins with SDS-PAGE or by a biological assay.

### Assay of Molecular Weight by SDS-PAGE

The exosome may have a molecular weight of greater than 100 kDa. For example, the exosome may be such that most proteins of the exosome with less than 100 kDa molecular weight segregate into the greater than 100 kDa molecular weight retentate fraction, when subject to filtration. Similarly, when subjected to filtration with a membrane with a 500 kDa cut off, most proteins of the exosome with less than 500 kDa molecular weight may segregate into the greater than 500 kDa molecular weight retentate fraction. This indicates that the exosome may have a molecular weight of more than 500 kDa.

### Assay of Molecular Weight by Biological Activity

The exosome may have a molecular weight of more than 1000 kDa. For example, the exosome may be such that when subject to filtration with a membrane with a molecular weight cutoff of 1000 kDa, the relevant biological activity substantially or predominantly remains in the retentate fraction. Alternatively or in addition, biological activity may be absent in the filtrate fraction. The biological activity may comprise any of the biological activities of the exosome described elsewhere in this document.

### Assay of Molecular Weight by Infarct Size

For example, the biological activity may comprise reduction of infarct size, as assayed in any suitable model of myocardia ischemia and reperfusion injury. For example, the biological activity may be assayed in a mouse or pig model, as described in WO 2009/105044.

In summary, myocardial ischemia is induced by 30 minutes left coronary artery (LCA) occlusion by suture ligation and reperfusion is initiated by removal of suture. Mice are treated with liquid containing the exosomes (such as unfractionated MSC-CM), filtrate (such as < 100 or 1,000 kD fraction), retentate (such as > 1000 kD retentate) or saline intravenously via the tail vein, 5 minutes before reperfusion. 24 hours later, the hearts are excised. Before excision, the Area At Risk (AAR) is determined by religating the LCA and then perfusing Evans blue through the aorta.

AAR is defined as the area not stained by the dye and is expressed as a percentage of the left ventricular wall area. Infarct size is assessed 24 hours later using Evans blue and TTC. Where the relative infarct size is significantly reduced in animals treated with mesenchymal stem cell conditioned medium (MSC-CM) and the retentate (such as a >1000 kD) fraction when compared to saline, this indicates that the exosome has a molecular weight which is higher than the relevant cutoff of the membrane (e.g., greater than 1000 kDa).

### Exosome Size

The exosome may have a size of greater than 2 nm. The exosome may have a size of greater than 5 nm, 10 nm, 20 nm, 30 nm, 40 nm or 50 nm. The exosome may have a size of greater than 100 nm, such as greater than 150 nm. The exosome may have a size of substantially 200 nm or greater.

The exosome may have a range of sizes, such as between 2 nm to 20 nm, 2 nm to 50 nm, 2 nm to 100 nm, 2 nm to 150 nm or 2 nm to 200 nm. The exosome may have a size between 20 nm to 50 nm, 20 nm to 100 nm, 20 nm to 150 nm or 20 nm to 200 nm. The exosome may have a size between 50 nm to 100ηm, 50ηm to 150 nm or 50 nm to 200 nm. The exosome may have a size between 100 nm to 150 nm or 100 nm to 200 nm. The exosome may have a size between 150 nm to 200 nm.

The size may be determined by various means. In principle, the size may be determined by size fractionation and filtration through a membrane with the relevant size cut-off. The exosome size may then be determined by tracking segregation of component proteins with SDS-PAGE or by a biological assay.

The size may also be determined by electron microscopy.

The size may comprise a hydrodynamic radius. The hydrodynamic radius of the exosome may be below 100 nm. It may be between about 30 nm and about 70 nm. The hydrodynamic radius may be between about 40 nm and about 60 nm, such as between about 45 nm and about 55 nm. The hydrodynamic radius may be about 50 nm.

The hydrodynamic radius of the exosome may be determined by any suitable means, for example, laser diffraction or dynamic light scattering. An example of a dynamic light scattering method to determine hydrodynamic radius is described in WO 2009/105044.

### OBTAINING MESENCHYMAL STEM CELLS (MSC)

Mesenchymal stem cell particles such as exosomes may be isolated or produced, using the methods described here, from mesenchymal stem cell conditioned medium (MSC-CM).

MSCs suitable for use in the production of conditioned media and exosomes may be made by any method known in the art.

In particular, MSCs may be made by propagating a cell obtained by dispersing a embryonic stem (ES) cell colony, or a descendent thereof, in the absence of co-culture in a serum free medium comprising FGF2. This is described in detail in the sections below.

Methods of obtaining mesenchymal stem cells (MSC) or MSC-like cells from hESCs may involve either transfection of a human telomerase reverse transcriptase (hTERT) gene into differentiating hESCs (Xu et al., 2004) or coculture with mouse OP9 cell line (Barberi et al., 2005). The use of exogenous genetic material and mouse cells in these derivation protocols introduces unacceptable risks of tumorigenicity or infection of xenozootic infectious agents.

The exosomes may therefore be made from MSCs derived by the use of a clinically relevant and reproducible protocol for isolating similar or identical (such as homogenous) MSC populations from differentiating hESCs. In general, the method comprises dispersing a embryonic stem (ES) cell colony into cells. The cells are then plated out and propagated. The cells are propagated in the absence of co-culture in a serum free medium comprising fibroblast growth factor 2 (FGF2), in order to obtain mesenchymal stem cells (MSCs).

Thus, the protocol does not require serum, use of mouse cells or genetic manipulations and requires less manipulations and time, and is therefore highly scalable. The protocol may be used for the isolation of MSCs from two different hESC lines, HuES9 and H-1 and also a third one, Hes-3. Human ES cell derived MSCs (hESC-MSCs) obtained by the methods and compositions described here are remarkably similar to bone-marrow derived MSCs (BM-MSCs).

The embryonic stem cell culture may comprise a human embryonic stem cell (hESC) culture.

In a one embodiment, a method of generating mesenchymal stem cells (MSC) comprises trypsinizing and propagating hESCs without feeder support in media supplemented with FGF2 and optionally PDGF AB before sorting for CD105+CD34- cells.

The method may comprise sorting for CD105+, CD34- cells from trypsinized hESCs one week after feeder-free propagation in a media supplemented with FGF2 and optionally PDGF AB will generate to generate a hESC-MSC cell culture in which at least some, such as substantially all, or all cells are similar or identical (such as homogenous) to each other.

The MSCs produced by this method may be used to produce mesenchymal stem cell conditioned medium (MSC-CM), from which the exosomes may be isolated.

### Disaggregating Embryonic Stem Cell Colonies

One method of producing mesenchymal stem cells may comprise dispersing or disaggregating an embryonic stem cell colony into cells.

The embryonic stem cell colony may comprise a huES9 colony (Cowan CA, Klimanskaya I, McMahon J, Atienza J, Witmyer J, et al. (2004) Derivation of embryonic stem-cell lines from human blastocysts. N Engl J Med 350: 1353-1356) or a H1 ESC colony (Thomson JA, Itskovitz-Eldor J, Shapiro SS, Waknitz MA, Swiergiel JJ, et al. (1998) Embryonic Stem Cell Lines Derived from Human Blastocysts. Science 282: 1145-1147.).

The cells in the colony may be disaggregated or dispersed to a substantial extent, i.e., at least into clumps. The colony may be disaggregated or dispersed to the extent that all the cells in the colony are single, i.e., the colony is completely disaggregated.

The disaggregation may be achieved with a dispersing agent.

The dispersing agent may be anything that is capable of detaching at least some embryonic stem cells in a colony from each other. The dispersing agent may comprise a reagent which disrupts the adhesion between cells in a colony, or between cells and a substrate, or both. The dispersing agent may comprise a protease.

The dispersing agent may comprise trypsin. The treatment with trypsin may last for example for 3 minutes or thereabouts at 37 degrees C. The cells may then be neutralised, centrifuged and resuspended in medium before plating out.

The method may comprise dispersing a confluent plate of human embryonic stem cells with trypsin and plating the cells out.

The disaggregation may comprise at least some of the following sequence of steps: aspiration, rinsing, trypsinization, incubation, dislodging, quenching , re-seeding and aliquoting. The following protocol is adapted from the Hedrick Lab, UC San Diego (http://hedricklab.ucsd.edu/Protocol/COSCell.html).

In the aspiration step, the media is aspirated or generally removed from the vessel, such as a flask. In the rinsing step, the cells are rinsed with a volume, for example 5-10 mls, of a buffered medium, which is may be free from Ca²⁺ and Mg²⁺. For example, the cells may be rinsed with calcium and magnesium free PBS. In the trypsinization step, an amount of dispersing agent in buffer is added to the vessel, and the vessel rolled to coat the growing surface with the dispersing agent solution. For example, 1 ml of trypsin in Hank's BSS may be added to a flask.

In the incubation step, the cells are left for some time at a maintained temperature. For example, the cells may be left at 37°C for a few minutes (e.g., 2 to 5 minutes). In the dislodging step, the cells may be dislodged by mechanical action, for example by scraping or by whacking the side of the vessel with a hand. The cells should come off in sheets and slide down the surface.

In the quenching step, a volume of medium is added to the flask. The medium may comprise a neutralising agent to stop the action of the dispersing agent. For example, if the dispersing agent is a protease such as trypsin, the medium may contain a protein, such as a serum protein, which will mop up the activity of the protease. In a particular example, 3 ml of serum containing cell culture medium is added to the flask to make up a total of 4 mls. The cells may be pipetted to dislodge or disperse the cells.

In the re-seeding step, the cells are re-seeded into fresh culture vessels and fresh medium added. A number of re-seedings may be made at different split ratios. For example, the cells may be reseeded at 1/15 dilution and 1/5 dilution. In a particular example, the cells may be re-seeded by adding 1 drop of cells into a 25cm² flask and 3 drops into another to re-seed the culture, and 7-8 mls media is then added to each to provide for 1/15 dilution and 1/5 dilution from for example a 75cm² flask. In the aliquoting step, the cells may be aliquoted into new dishes or whatever split ratio is desired, and media added.

In a specific embodiment, the method includes the following steps: human ES cells are first grown suspended in non-adherent manner to form embryoid bodies (EBs). 5-10 day old EBs are then trypsinized before plating as adherent cells on gelatine coated tissue culture plates.

### Maintenance as Cell Culture

The disaggregated cells may be plated and maintained as a cell culture.

The cells may be plated onto a culture vessel or substrate such as a gelatinized plate. Crucially, the cells are grown and propagated without the presence of co-culture, e.g., in the absence of feeder cells.

The cells in the cell culture may be grown in a serum-free medium which is supplemented by one or more growth factors such as fibroblast growth factor 2 (FGF2) and optionally platelet-derived growth factor AB (PDGF AB), at for example 5ng/ml. The cells in the cell culture may be split or subcultured 1:4 when confluent, by treatment with trypsin, washing and replating.

### Absence of Co-Culture

The cells may be cultured in the absence of co-culture. The term "co-culture" refers to a mixture of two or more different kinds of cells that are grown together, for example, stromal feeder cells.

Thus, in typical ES cell culture, the inner surface of the culture dish is usually coated with a feeder layer of mouse embryonic skin cells that have been treated so they will not divide. The feeder layer provides an adherent surface to enable the ES cells to attach and grow. In addition, the feeder cells release nutrients into the culture medium which are required for ES cell growth. In the methods and compositions described here, the ES and MSC cells may be cultured in the absence of such co-culture.

The cells may be cultured as a monolayer or in the absence of feeder cells. The embryonic stem cells may be cultured in the absence of feeder cells to establish mesenchymal stem cells (MSC).

The dissociated or disaggregated embryonic stem cells may be plated directly onto a culture substrate. The culture substrate may comprise a tissue culture vessel, such as a Petri dish. The vessel may be pre-treated. The cells may be plated onto, and grow on, a gelatinised tissue culture plate.

An example protocol for the gelatin coating of dishes follows. A solution of 0.1% gelatin in distilled water is made and autoclaved. This may be stored at room temp. The bottom of a tissue culture dish is covered with the gelatin solution and incubated for 5-15min. Remove gelatin and plates are ready to use. Medium should be added before adding cells to prevent hypotonic lysis.

### Serum Free Media

The dissociated or disaggregated embryonic stem cells may be cultured in a medium which may comprise a serum-free medium.

The term "serum-free media" may comprise cell culture media which is free of serum proteins, e.g., fetal calf serum. Serum-free media are known in the art, and are described for example in US Patents 5,631,159 and 5,661,034. Serum-free media are commercially available from, for example, Gibco-BRL (Invitrogen).

The serum-free media may be protein free, in that it may lack proteins, hydrolysates, and components of unknown composition. The serum-free media may comprise chemically-defined media in which all components have a known chemical structure. Chemically-defined serum-free media is advantageous as it provides a completely defined system which eliminates variability allows for improved reproducibility and more consistent performance, and decreases possibility of contamination by adventitious agents.

The serum-free media may comprise Knockout DMEM media (Invitrogen-Gibco, Grand Island, New York).

The serum-free media may be supplemented with one or more components, such as serum replacement media, at a concentration of for example, 5%, 10%, 15%, etc. The serum-free media may comprise or be supplemented with 10% serum replacement media from Invitrogen- Gibco (Grand Island, New York).

### Growth Factor

The serum-free medium in which the dissociated or disaggregated embryonic stem cells are cultured may comprise one or more growth factors. A number of growth factors are known in the art, including PDGF, EGF, TGF-a, FGF, NGF, Erythropoietin, TGF-b, IGF-I and IGF-II.

The growth factor may comprise fibroblast growth factor 2 (FGF2). The medium may also contain other growth factors such as platelet-derived growth factor AB (PDGF AB). Both of these growth factors are known in the art. The method may comprise culturing cells in a medium comprising both FGF2 and PDGF AB.

Alternatively, or in addition, the medium may comprise or further comprise epidermal growth factor (EGF). Use of EGF may enhance growth of MSCs. EGF may be used at any suitable concentration, for example 5-10 ng/ml EGF. EGF may be used in place of PDGF. EGF is a protein well known in the art, and is referred to as symbol EGF, Alt. Symbols URG, Entrez 1950, HUGO 3229, OMIM 131530, RefSeq NM_001963, UniProt P01133.

Thus, we disclose the use of media comprising (i) FGF2, (ii) FGF2 and PDGF and (iii) FGF2 and EGF and other combinations.

FGF2 is a wide-spectrum mitogenic, angiogenic, and neurotrophic factor that is expressed at low levels in many tissues and cell types and reaches high concentrations in brain and pituitary. FGF2 has been implicated in a multitude of physiologic and pathologic processes, including limb development, angiogenesis, wound healing, and tumor growth. FGF2 may be obtained commercially, for example from Invitrogen-Gibco (Grand Island, New York).

Platelet Derived Growth Factor (PDGF) is a potent mitogen for a wide range of cell types including fibroblasts, smooth muscle and connective tissue. PDGF, which is composed of a dimer of two chains termed the A chain and B chain, can be present as AA or BB homodimers or as an AB heterodimer. Human PDGF-AB is a 25.5 kDa homodimer protein consisting of 13.3 kDa A chain and 12.2 B chain. PDGF AB may be obtained commercially, for example from Peprotech (Rocky Hill, New Jersey).

The growth factor(s), such as FGF2 and optionally PDGF AB, may be present in the medium at concentrations of about 100pg/ml, such as about 500pg/ml, such as about 1ng/ml, such as about 2ng/ml, such as about 3ng/ml, such as about 4ng/ml, such as about 5ng/ml. In some embodiments, the medium contains FGF2 at about 5ng/ml. The medium may also contain PDGF AB, such as at about 5ng/ml.

### Splitting Cells

Cells in culture will generally continue growing until confluence, when contact inhibition causes cessation of cell division and growth. Such cells may then be dissociated from the substrate or flask, and "split", subcultured or passaged, by dilution into tissue culture medium and replating.

The methods and compositions described here may therefore comprise passaging, or splitting during culture. The cells in the cell culture may be split at a ratio of 1:2 or more, such as 1:3, such as 1:4, 1:5 or more. The term "passage" designates the process consisting in taking an aliquot of a confluent culture of a cell line, in inoculating into fresh medium, and in culturing the line until confluence or saturation is obtained.

### Selection, Screening or Sorting Step

The method may further comprise a selection or sorting step, to further isolate or select for mesenchymal stem cells.

The selection or sorting step may comprise selecting mesenchymal stem cells (MSC) from the cell culture by means of one or more surface antigen markers. The use of a selection or sorting step further enhances the stringency of sorting and selection specificity for MSCs and furthermore potentially reduces possible contamination from embryonic stem cells such as hESCs and other hESC-derivatives from the starting material. This would then further reduce the risk of teratoma formation and further increase the clinical relevance of the protocol we describe.

A number of methods are known for selection or sorting based on antigen expression, and any of these may be used in the selection or sorting step described here. The selection or sorting may be achieved by means of fluorescence activated cell sorting (FACS). Thus, as known in the art, FACS involves exposing cells to a reporter, such as a labelled antibody, which binds to and labels antigens expressed by the cell. Methods of production of antibodies and labelling thereof to form reporters are known in the art, and described for example in Harlow and Lane. The cells are then passed through a FACS machine, which sorts the cells from each other based on the labelling. Alternatively or in addition, magnetic cell sorting (MACS) may be employed to sort the cells.

We have realised that while a number of candidate surface antigens known to be associated with MSCs e.g. CD105, CD73, ANPEP, ITGA4 (CD49d), PDGFRA, some of the MSC associated surface antigens e.g. CD29 and CD49e are also highly expressed in ES cells such as hESCs and their expression are verified by FACS analysis. The association of a surface antigen with MSCs may not be sufficient to qualify the antigen as a selectable marker for isolating MSCs from ES cells such as hESC. Accordingly, the selection or sorting step may employ antigens which are differentially expressed between MSCs and ES cells.

The selection or sorting step of our method may positively select for mesenchymal stem cells based on the expression of antigens. Such antigens may be identified by, for example, comparing the gene expression profiles of hESCs and hESCMSCs.

The selection or sorting step of our method may positively select for mesenchymal stem cells based on the expression of antigens which are identified as expressed on MSCs, but not expressed on ES cells such as hESCs.

CD73 is highly expressed on MSCs, while being not highly expressed on hESCs. Both CD73 and CD105 are highly expressed surface antigens in MSCs and are among the top 20 highly expressed surface antigens in hESC-MSCs relative to hESC, the use of either CD73 or CD105 (or both) as selectable marker for putative MSCs will be equally effective in sorting for putative MSCs generated by differentiating hESCs.

Alternatively, or in addition, the selection or sorting step may negatively select against antigens based on surface antigens that are highly expressed as surface antigen on embryonic stem cells (ES cells) such as hESCs, and not mesenchymal stem cells e.g., hESC-MSC. Selection or sorting may be based on known or previously identified hESC-specific surface antigens such as MIBP, ITGB1BP3 and PODXL, and CD34.

FACS analysis confirms the expression of CD34 on hESC but not hESC-MSCs. Therefore, CD34 may be used as a negative selection or sorting marker either on its own, or in conjunction with CD105 as a positive selectable marker for isolating putative MSCs from differentiating hESC cultures.

### EXOSOME PREPARATION

Exosomes may be prepared according to the methods disclosed in International Patent Publication Number WO 2009/105044.

Ion exchange chromatography, as described in International Patent Publication WO 2012/087241, may also be used to separate and/or purify exosomes from mesenchymal stem cells.

### Example Protocol (Ion Exchange Chromatography)

The following paragraphs provide a specific example of how a mesenchymal stem cell exosome may be obtained using ion exchange chromatography.

A mesenchymal stem cell exosome may be produced by culturing mesenchymal stem cells in a medium to condition it. The mesenchymal stem cells may comprise HuES9.E1 cells. The medium may comprise DMEM. The DMEM may be such that it does not comprise phenol red. The medium may be supplemented with insulin, transferrin, or selenoprotein (ITS), or any combination thereof. It may comprise FGF2. It may comprise PDGF AB. The concentration of FGF2 may be about 5 ng/ml FGF2. The concentration of PDGF AB may be about 5 ng/ml. The medium may comprise glutamine-penicillin-streptomycin or β-mercaptoethanol, or any combination thereof.

The cells may be cultured for about 1, 2, 3, 4, 5, 6, 7, 8, 9, 10 days or more, for example 3 days. The conditioned medium may be obtained by separating the cells from the medium. The conditioned medium may be centrifuged, for example at 500 g. it may be concentrated by filtration through a membrane. The membrane may comprise a > 1000 kDa membrame. The conditioned medium may be concentrated about 50 times or more.

The conditioned medium may then be subjected to anion exchange chromatography, as described in the Examples.

In addition to detecting the alpha subunit of the 20S proteasome and CD9, UV absorbance such as at 220 nm may also be used to track the progress of elution. Fractions may be examined for dynamic light scattering (DLS) using a quasi-elastic light scattering (QELS) detector.

Fractions which are found to exhibit dynamic light scattering may be retained. In addition to anion exchange chromatography, the conditioned medium may be separated by size exclusion, as an additional step. Any size exclusion matrix such as Sepharose may be used. As an example, a TSK Guard column SWXL, 6 × 40 mm or a TSK gel G4000 SWXL, 7.8 × 300 mm may be employed. The eluent buffer may comprise any physiological medium such as saline. It may comprise 20 mM phosphate buffer with 150 mM of NaCl at pH 7.2. The chromatography system may be equilibrated at a flow rate of 0.5 ml/min. The elution mode may be isocratic.

For example, a fraction which is produced by the general method as described above, and which elutes with a retention time of 11-13 minutes, such as 12 minutes, is found to exhibit dynamic light scattering. The rₕ of exosomes in this peak is about 45-55 nm. Such fractions comprise mesenchymal stem cell exosomes.

### UTILITY

The methods and compositions described here enable the long term storage of exosomes and the preservation of their activity. The lyophilised exosome prepared using the methods described here may be used for a number of therapeutic purposes, as described below.

As the lyophilised exosome retain its activity through prolonged periods at elevated temperatures, the methods described here allow the exosomes to be easily stored, shipped, and later reconstituted to its original form for use, for example in the field.

### DISEASES TREATABLE BY PARTICLES FROM MESENCHYMAL STEM CELLS

Analysis of the proteome of MSCs shows that the proteins expressed are involved in three biological processes: metabolism, defense response, and tissue differentiation including vascularization, hematopoiesis and skeletal development.

Accordingly, the particles from MSCs such as exosomes purified as described here may be used to treat diseases which these functions may have a role in, or whose repair or treatment involves any one or more of these biological processes. Similarly, the proteins expressed by the MSCs, singly or in combination, preferably in the form of particles as described here, may be used to supplement the activity of, or in place of, the MSCs, or media conditioned by the MSCs, for the purpose of for example treating or preventing such diseases.

The gene products expressed by the MSCs are shown to activate important signalling pathways in cardiovascular biology, bone development and hematopoiesis such as Jak-STAT, MAPK, Toll-like receptor, TGF-beta signalling and mTOR signaling pathways. Accordingly, the particles from the MSCs, etc, may be used to prevent or treat a disease in which any of these signalling pathways is involved, or whose aetiology involves one or more defects in any one or more of these signalling pathways.

Accordingly, such particles such as exosomes purified as described here may be used to treat cardiac failure, bone marrow disease, skin disease, burns and degenerative diseases such as diabetes, Alzheimer's disease, Parkinson's disease and cancer.

Such particles such as exosomes purified as described here may also be used to treat myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease.

The particles such as exosomes purified as described here may be used to aid wound healing, scar reduction, bone formation, a bone graft or bone marrow transplantation in an individual.

Unless the context dictates otherwise, the term "conditioned medium" should be taken to include not only cell culture medium exposed to MSCs as well as such a composition comprising one or more, preferably substantially all, the polypeptides which are present in the conditioned medium.

The particles such as exosomes purified as described here may also be used as sources for any of the proteins secreted or expressed by the MSCs. We therefore provide for a method of producing a polypeptide as shown, the method comprising obtaining a particle such as an exosome purified as described here, and isolating the polypeptide from the particle.

### HEART DISEASE

The mesenchymal stem cell particle such as exosomes purified as described here may be used for treatment or prevention of heart disease.

Heart disease is an umbrella term for a variety for different diseases affecting the heart. As of 2007, it is the leading cause of death in the United States, England, Canada and Wales, killing one person every 34 seconds in the United States alone. Heart disease includes any of the following.

### CORONARY HEART DISEASE

Coronary artery disease is a disease of the artery caused by the accumulation of atheromatous plaques within the walls of the arteries that supply the myocardium. Angina pectoris (chest pain) and myocardial infarction (heart attack) are symptoms of and conditions caused by coronary heart disease. Over 459,000 Americans die of coronary heart disease every year. In the United Kingdom, 101,000 deaths annually are due to coronary heart disease.

### CARDIOMYOPATHY

Cardiomyopathy is the deterioration of the function of the myocardium (i.e., the actual heart muscle) for any reason. People with cardiomyopathy are often at risk of arrhythmia and/or sudden cardiac death. Extrinsic cardiomyopathies - cardiomyopathies where the primary pathology is outside the myocardium itself comprise the majority of cardiomyopathies . By far the most common cause of a cardiomyopathy is ischemia.

The World Health Organization includes as specific cardiomyopathies: Alcoholic cardiomyopathy, coronary artery disease, congenital heart disease, nutritional diseases affecting the heart, ischemic (or ischaemic) cardiomyopathy, hypertensive cardiomyopathy, valvular cardiomyopathy, inflammatory cardiomyopathy.

Also included are:
Cardiomyopathy secondary to a systemic metabolic disease
Intrinsic cardiomyopathies (weakness in the muscle of the heart that is not due to an identifiable external cause)
Dilated cardiomyopathy (DCM, the most common form, and one of the leading indications for heart transplantation. In DCM the heart (especially the left ventricle) is enlarged and the pumping function is diminished)
Hypertrophic cardiomyopathy (HCM or HOCM, a genetic disorder caused by various mutations in genes encoding sarcomeric proteins. In HCM the heart muscle is thickened, which can obstruct blood flow and prevent the heart from functioning properly),
Arrhythmogenic right ventricular cardiomyopathy (ARVC, which arises from an electrical disturbance of the heart in which heart muscle is replaced by fibrous scar tissue. The right ventricle is generally most affected)
Restrictive cardiomyopathy (RCM, which is the least common cardiomyopathy. The walls of the ventricles are stiff, but may not be thickened, and resist the normal filling of the heart with blood).

Noncompaction Cardiomyopathy - the left ventricle wall has failed to properly grow from birth and such has a spongy appearance when viewed during an echocardiogram.

### CARDIOVASCULAR DISEASE

Cardiovascular disease is any of a number of specific diseases that affect the heart itself and/or the blood vessel system, especially the veins and arteries leading to and from the heart. Research on disease dimorphism suggests that women who suffer with cardiovascular disease usually suffer from forms that affect the blood vessels while men usually suffer from forms that affect the heart muscle itself. Known or associated causes of cardiovascular disease include diabetes mellitus, hypertension, hyperhomocysteinemia and hypercholesterolemia.

Types of cardiovascular disease include atherosclerosis

### ISCHAEMIC HEART DISEASE

Ischaemic heart disease is disease of the heart itself, characterized by reduced blood supply to the organs. This occurs when the arteries that supply the oxygen and the nutrients gets stopped and the heart will not get enough of the oxygen and the nutrients and will eventually stop beating.

### HEART FAILURE

Heart failure, also called congestive heart failure (or CHF), and congestive cardiac failure (CCF), is a condition that can result from any structural or functional cardiac disorder that impairs the ability of the heart to fill with or pump a sufficient amount of blood throughout the body. Cor pulmonale is a failure of the right side of the heart.

### HYPERTENSIVE HEART DISEASE

Hypertensive heart disease is heart disease caused by high blood pressure, especially localised high blood pressure. Conditions that can be caused by hypertensive heart disease include: left ventricular hypertrophy, coronary heart disease, (Congestive) heart failure, hypertensive cardiomyopathy, cardiac arrhythmias, inflammatory heart disease, etc.

Inflammatory heart disease involves inflammation of the heart muscle and/or the tissue surrounding it. Endocarditis comprises inflammation of the inner layer of the heart, the endocardium. The most common structures involved are the heart valves. Inflammatory cardiomegaly. Myocarditis comprises inflammation of the myocardium, the muscular part of the heart.

### VALVULAR HEART DISEASE

Valvular heart disease is disease process that affects one or more valves of the heart. The valves in the right side of the heart are the tricuspid valve and the pulmonic valve. The valves in the left side of the heart are the mitral valve and the aortic valve. Included are aortic valve stenosis, mitral valve prolapse and valvular cardiomyopathy.

[The above text is adapted from Heart disease. (2009, February 3). In Wikipedia, The Free Encyclopedia. Retrieved 06:33, February 20, 2009, from http://en.wikipedia.org/w/index.php?title=Heart_disease&oldid=268290924]

### TISSUE REGENERATION

Mesenchymal stem cells particles isolated according to the methods and compositions described here may also be used for tissue reconstitution or regeneration in a human patient in need thereof. The particles such as exosomes may be administered in a manner that permits them to graft to the intended tissue site and reconstitute or regenerate the functionally deficient area.

For example, the methods and compositions described here may be used to modulate the differentiation of stem cells. Mesenchymal stem cell particles such as exosomes purified as described here therefrom may be used for tissue engineering, such as for the growing of skin grafts. Modulation of stem cell differentiation may be used for the bioengineering of artificial organs or tissues, or for prosthetics, such as stents.

### CANCER

Mesenchymal stem cell particles such as exosomes purified as described here may be used for the treatment of cancer.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia.

More particular examples of such cancers include squamous cell cancer, small-cell lung cancer, non-small cell lung cancer, gastric cancer, pancreatic cancer, glial cell tumors such as glioblastoma and neurofibromatosis, cervical cancer, ovarian cancer, liver cancer, bladder cancer, hepatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer. Further examples are solid tumor cancer including colon cancer, breast cancer, lung cancer and prostrate cancer, hematopoietic malignancies including leukemias and lymphomas, Hodgkin's disease, aplastic anemia, skin cancer and familiar adenomatous polyposis. Further examples include brain neoplasms, colorectal neoplasms, breast neoplasms, cervix neoplasms, eye neoplasms, liver neoplasms, lung neoplasms, pancreatic neoplasms, ovarian neoplasms, prostatic neoplasms, skin neoplasms, testicular neoplasms, neoplasms, bone neoplasms, trophoblastic neoplasms, fallopian tube neoplasms, rectal neoplasms, colonic neoplasms, kidney neoplasms, stomach neoplasms, and parathyroid neoplasms. Breast cancer, prostate cancer, pancreatic cancer, colorectal cancer, lung cancer, malignant melanoma, leukaemia, lympyhoma, ovarian cancer, cervical cancer and biliary tract carcinoma are also included.

The mesenchymal stem cell particles such as exosomes purified as described here may also be used in combination with anticancer agents such as endostatin and angiostatin or cytotoxic agents or chemotherapeutic agent. For example, drugs such as such as adriamycin, daunomycin, cis-platinum, etoposide, taxol, taxotere and alkaloids, such as vincristine, and antimetabolites such as methotrexate. The term "cytotoxic agent" as used herein refers to a substance that inhibits or prevents the function of cells and/or causes destruction of cells. The term is intended to include radioactive isotopes (e.g. I, Y, Pr), chemotherapeutic agents, and toxins such as enzymatically active toxins of bacterial, fungal, plant or animal origin, or fragments thereof.

Also, the term includes oncogene product/tyrosine kinase inhibitors, such as the bicyclic ansamycins disclosed in WO 94/22867; 1,2-bis(arylamino) benzoic acid derivatives disclosed in EP 600832; 6,7-diamino-phthalazin-1-one derivatives disclosed in EP 600831; 4,5-bis(arylamino)-phthalimide derivatives as disclosed in EP 516598; or peptides which inhibit binding of a tyrosine kinase to a SH2-containing substrate protein (see WO 94/07913, for example). A "chemotherapeutic agent" is a chemical compound useful in the treatment of cancer. Examples of chemotherapeutic agents include Adriamycin, Doxorubicin, 5-Fluorouracil (5-FU), Cytosine arabinoside (Ara-C), Cyclophosphamide, Thiotepa, Busulfan, Cytoxin, Taxol, Methotrexate, Cisplatin, Melphalan, Vinblastine, Bleomycin, Etoposide, Ifosfamide, Mitomycin C, Mitoxantrone, Vincristine, VP-16, Vinorelbine, Carboplatin, Teniposide, Daunomycin, Carminomycin, Aminopterin, Dactinomycin, Mitomycins, Nicotinamide, Esperamicins (see U.S. Pat. No. 4,675,187), Melphalan and other related nitrogen mustards, and endocrine therapies (such as diethylstilbestrol (DES), Tamoxifen, LHRH antagonizing drugs, progestins, anti-progestins etc).

### EXAMPLES

### Example 1. Materials and Methods

200 µL 5 × lyophilization buffer was added to 1 mg exosome in 800 µL PBS and mixed. 50 µL of the solution was then aliquoted into each 1.5ml microcentrifuge tube. The tube was capped and 2 holes were punched on the cap of each tube using an 18 gauge needle. The sample was frozen at -20°C for 3 hours. Transfer frozen samples to freeze dryer pre-cooled to -48°C. Pressure in the freeze-dryer chamber was maintained at < 0.05mbar for about 12-24 hours. When completed, the tubes are removed and place tubes in a dessicator for several minutes until temperature has reached room temperature. Seal the holes on the cap using a parafilm and store samples in a desiccator at -20°C, 4°C, 20°C or 40°C. Alternatively, the lyophilized exosomes should be stored under a dry inert gas such as nitrogen in air-tight vials.

**Table E1. 1 × Lyophilization buffer recipe**

| | **Component** | **Preferred ingredients** |
|---|---|---|
| 1 | Buffer, pH6.5-7.5 | Potassium phosphate buffer: 0.00376M KH₂PO₄ and 0.0071M K₂HPO₄, pH 7.0 |
| 2 | di-saccharide | 4 % trehalose (w/v) (alternative dissacharides : sorbitol, sucrose); preferred concentration < 10% w/v |
| 3 | Amino acid | 0.0049M L-gulatamic acid (optional) |
| 4 | Protein binding stabiliser | 1% human serum albumin (w/v): highly optional |

### Example 2. Stability of Exosome-Associated CD73 Enzyme in Lyophilized Exosomes at 40°C

Lyophilized exosomes as prepared according to the lyophilization protocol above were stored at 40°C to assess their thermal stability.

CD73, which is associated with exosomes, is used as a surrogate marker for exosome stability.

CD73 enzymatic activity is a biomarker of exosome therapeutic activity²¹. MSC exosomes in PBS were lyophilized with or without the lyophilization buffer. The lyophilized exosomes were stored at 40°C and assayed for CD73 activity at various times. CD73 activity in exosomes was measured by incubating 2.5 µg of exosomes in 100 µL Tris buffer pH 7.4 containing 50 µM AMP (Sigma-Aldrich, St. Louis, MO) and measuring the amount of phosphate ions released from the hydrolysis of AMP by Colorlock Gold kit (Innova Biosciences, Cambridge, UK).²¹ CD73 activity was normalized to the activity of exosome lyophilized in the lyophilization buffer on day 1 of storage at 40°C.

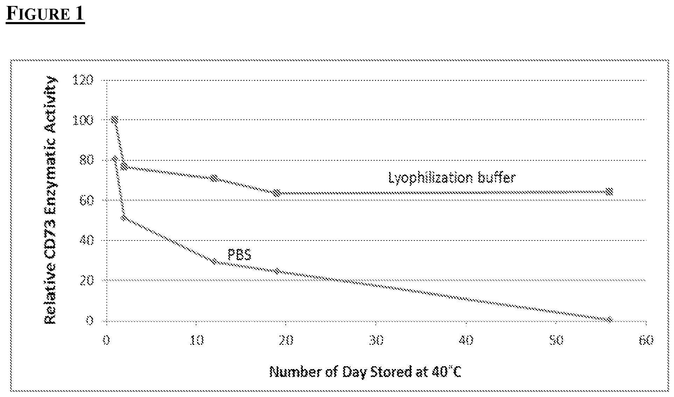

Storage of the lyophilized exosomes resulted in an immediate precipitous loss of 20% and 40% in CD73 activity in exosomes lyophilized with the lyophilization buffer or PBS, respectively (Figure 1).

Use of lyophilization buffer attenuated the decay of CD73 enzymatic activity such that the residual activity on day 55 of storage at 40°C was about 60% of the starting activity.

However, lyophilization of the exosomes in PBS not only reduced activity to about 80% of that in lyophilization buffer on day 1, it also led to a linear loss of activity such that no activity was detected on day 55.

We noted that the use of sugars at > 10% (w/v) will cause Malliard reaction when the lyophilized exosomes are stored for prolonged periods at >0°C. This evidenced by the browning of the lyophilized exosomes.

### Example 3. Preservation of Exosome Number and Size after Lyophilization

To determine if lyophilization affect the number and size of exosome after lyophilization, the number and size of lyophilized exosomes were compared to those of exosomes stored frozen at -20°C.

Four exosome samples at 1 mg/ml concentration were prepared:
1) exosome in PBS;
2) exosomes in lyophilization buffer;
3) exosomes in lyophilization buffer where 4% (w/v) trehalose was replaced with 4% (w/v) sucrose;
4) exosomes in lyophilization buffer where 4% (w/v) trehalose was replaced with 4% (w/v) sorbitol.

Each sample was split into two aliquots as "before" and "after" lyophilization samples.

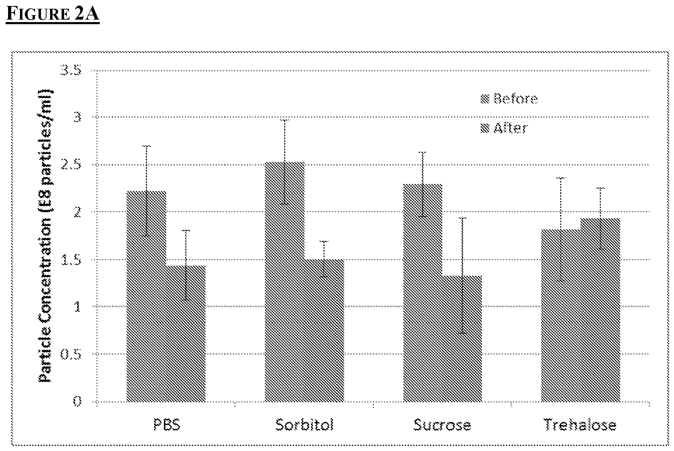

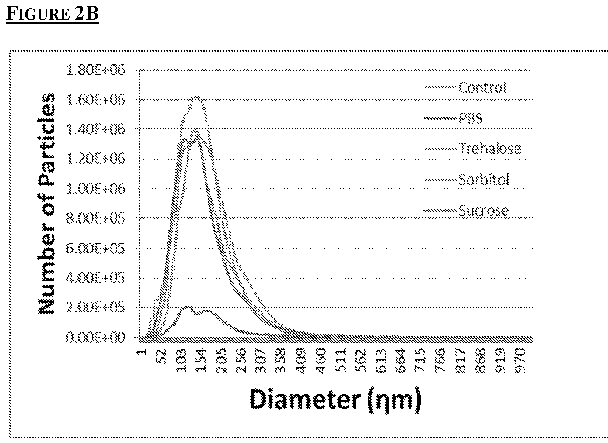

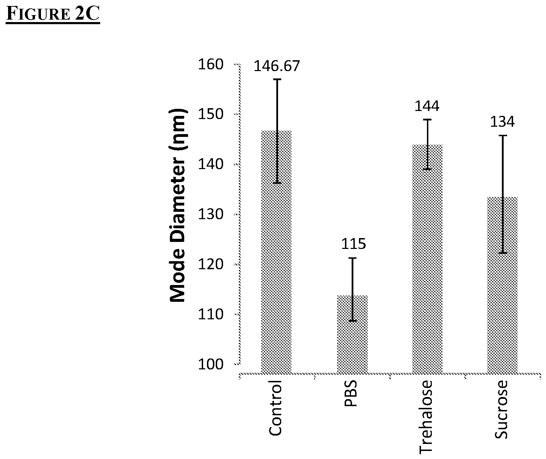

The results are shown in Figure 2A, Figure 2B and Figure 2C.

Figure 2A. Four exosome samples at 1 mg/ml concentration were prepared 1) exosome in PBS; 2) exosomes in lyophilization buffer; 3) exosomes in lyophilization buffer where 4% (w/v) trehalose was replaced with 4% (w/v) sucrose; 4) exosomes in lyophilization buffer where 4% (w/v) trehalose was replaced with 4% (w/v) sorbitol. Each sample was split into two aliquots as "before" and "after" lyophilization samples. The "after" samples were reconstituted with water and the volume of water was that of the buffer in the "before" sample. The number of particles in the "before" and "after" were measured using NanoSight LM10 coupled with a 405-nm laser (Malvern, Worcestershire, UK) and analysed by Nanoparticle Tracking Analysis (NTA)2.3 software (Malvern, Worcestershire, UK) as previously reported ²². Each of the samples was diluted to 2 µg protein/mL with 0.22 mm filtered PBS and loaded using the NanoSight syringe pump and script control system set at 100 arbitrary units as recommended by NanoSight and analyzed as previously described²².

Figure 2B and Figure 2C. The size distribution and modal diameter of the particles in each of the reconstituted lyophilized samples was analyzed using NanoSight NTA against the original exosome preparation that had been stored at -80°C. Figures 2B and 2C. The size distribution and modal diameter of the particles in each of the reconstituted lyophilized samples was analyzed using NanoSight NTA against the original exosome preparation that had been stored at -80°C.

Of the four different lyophilization buffers, trehalose preserved the highest number of particles before and after lyophilisation (Figure 2A).

All three di-saccharides were equally effective in maintaining the size distibution of the particles of the original exosome (Figure 2B).

However, sucrose was not as effective in preserving the modal diameter of the exosomes (Figure 2C).

### Example 4. Cardioprotection by Exosomes Lyophilized with Trehalose versus Sucrose

To determine if the better preservation of particle number during lyophilization by trehalose *versus* sucrose translates into better preservation of therapeutic potency, the cardioprotection potency of exosomes lyophilized in either trehalose (exo Tre) or sucrose (exo Suc) was assessed in an *ex vivo* Landerdorf model of ischemia-reperfusion injury.

In this model, cardioprotection is measured by the degree of hypercontracture over post-reperfusion time.

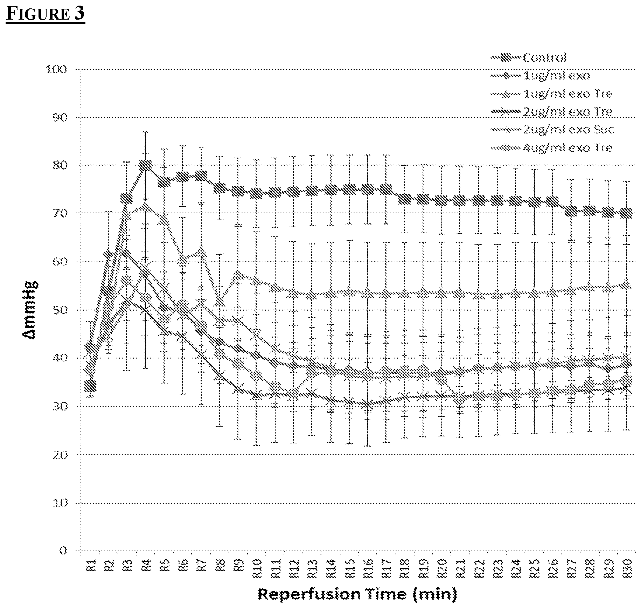

The results are shown in Figure 3.

Exosomes were tested for their efficacy to limit reperfusion injury in an *ex vivo* Langendorff model of isolated rat heart perfusion. In this model, global ischemia was induced by stopping perfusion for 30 min and the heart was then re-perfused with the perfusion buffer (control) or perfusion buffer with exosomes. The exosomes used were either exosomes stored at - 80°C for less than 3 months at the time of analysis (exo), reconstituted lyophilized exosomes that were lyophilized in the presence of trehalose (exo Tre) or sucrose (exo, Suc). The heart function was evaluated by the degree of hypercontraction which is the difference between the average minimum LVDP at reperfusion time R and average minimum LVDP one minute before ischemia. Each data point was the average of five hearts.

The hypercontracture for 2 µg exo Tre was reduced relative to that using 2 µg exo Suc, demonstrating that Trehalose preserves therapeutic potency of exosomes.

### Example 5. Stability of the Biological Potency of Lyophilized Exosome

To assess the stability of the biological potency of lyophilized exosomes, the lyophilized exosomes were either stored at 4°C or 40°C for 3 days, 1 week and 3weeks, and then tested for their potency to induce polarization of CD4+ T cells to regulatory T cells (Tregs).

This potency was compared to non-lyophilized exosomes that had been stored at -80°C for less than 3 months at the time of analysis.

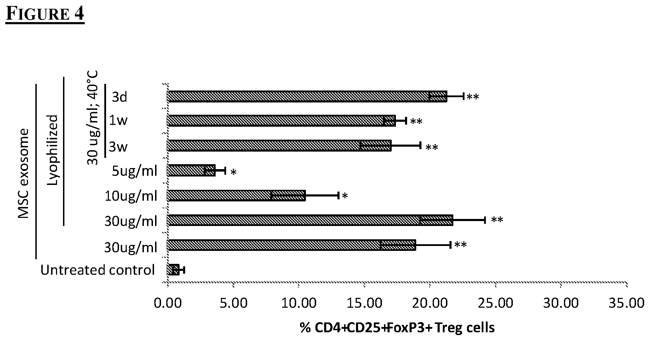

The results are shown in Figure 4.

Allogeneic CD11c+ cells from BALB/c mouse spleens were incubated with CD4+ T cells from C57BL/6 mouse spleens at a cell ratio of 1:5 in the presence of frozen or lyophilized MSC exosomes. The untreated control referred to the incubation of CD11c+ cells and CD4+ T cells without exosomes. The frozen exosomes were stored at -80°C for < 3 months at the time of the experiment. The lyophilized exosomes were stored at 4°C for a week at the time of analysis or at 40°C for 3days (d), 1week (w) or 3 weeks (w). After 6 days, the cells were harvested and analyzed by FACS for the presence of CD4+CD25+Foxp3+ Treg cells. Each bar represents the mean (± SD) of 1 assay performed in triplicate. * p < 0.05, **p < 0.001.

Relative to non-lyophilized exosomes stored at -80°C, lyophilized exosomes stored at 4°C and 40°C was just as potent (Figure 4).

### Example 6. Loading of Exosomes on Medical Devices by Freeze Drying Followed by Sterilization via Ionizing Radiation of Exosomes

As proof of principle, biomaterial scaffolds such as collagen membrane were soaked with exosomes in the freeze-dry formulation, and the soaked membrane was lyophilized.

Some of the lyophilized material was irradiated with 25 Gray γ irradiation.

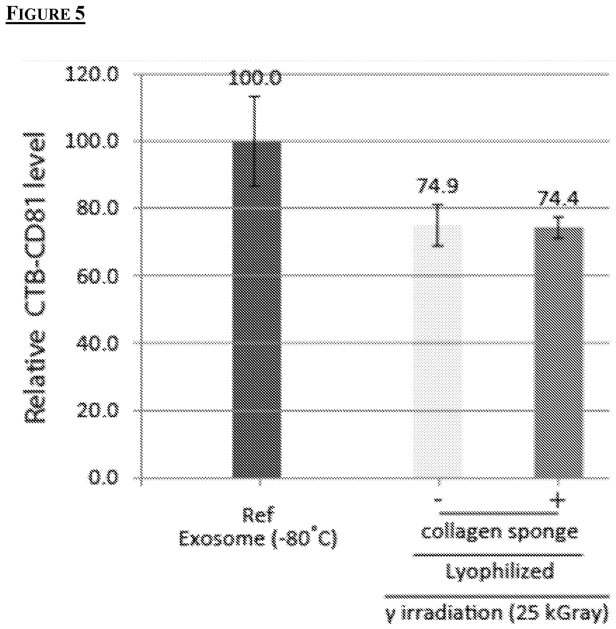

Water was then added in a volume equivalent to the volume of freeze-dry exosome-collagen membrane and pressure was applied to the membrane with a syringe plunger to extract the water. An aliquot was assayed for intact exosomes using CTB-CD81 (Figure 5).

A collagen membrane was soaked with exosomes in the freeze-dry formulation, and the soaked membrane was lyophilized. Some of the lyophilized material was also irradiated with 25 Gray γ irradiation. Water was then added in a volume equivalent to the volume of freeze-dry exosome-collagen membrane and pressure was applied to the membrane with a syringe plunger to extract the water. An aliquot was assayed for intact exosomes using CTB-CD81.

Freeze drying of exosomes with biomaterial scaffolds allowed the exosomes to be incorporated in the materials.

The exosomes can be easily eluted from the scaffolds by adding water (Figure 5).

Gamma sterilization, which is known to cause protein degradation²³, did not cause significant loss of exosome integrity using a previously described CTB-CD81 assay as previously described²⁴.

### REFERENCES

1. Vrijsen, K.R., et al. Cardiomyocyte progenitor cell-derived exosomes stimulate migration of endothelial cells. J Cell Mol Med 14, 1064-1070 (2010).
2. Barutta, F., et al. Urinary exosomal microRNAs in incipient diabetic nephropathy. PLoS ONE 8, e73798 (2013).
3. Pittenger, M., et al. Multilineage potential of adult human mesenchymal stem cells. Science 284, 143 - 147 (1999).
4. Deans, R.J. & Moseley, A.B. Mesenchymal stem cells. Experimental hematology 28, 875-884.
5. Hass, R. & Otte, A. Mesenchymal stem cells as all-round supporters in a normal and neoplastic microenvironment. Cell Commun Signal 10, 26 (2012).
6. Dominici, M. Minimal criteria for defining multipotent mesenchymal stromal cells. The International Society for Cellular Therapy position statement. Cytotherapy 8, 315-317 (2006).
7. Hass, R., Kasper, C., Bohm, S. & Jacobs, R. Different populations and sources of human mesenchymal stem cells (MSC): A comparison of adult and neonatal tissue-derived MSC. Cell Communication and Signaling 9, 12 (2011).
8. Ehninger, A. & Trumpp, A. The bone marrow stem cell niche grows up: mesenchymal stem cells and macrophages move in. The Journal of Experimental Medicine 208, 421-428 (2011).
9. Droujinine, I.A., Eckert, M.A. & Zhao, W. To grab the stroma by the horns: From biology to cancer therapy with mesenchymal stem cells, (2013).
10. Giordano, A., Galderisi, U. & Marino, I.R. From the laboratory bench to the patient's bedside: An update on clinical trials with mesenchymal stem cells. Journal of Cellular Physiology 211, 27-35 (2007).
11. Caplan, A.I. & Dennis, J.E. Mesenchymal stem cells as trophic mediators. J Cell Biochem 98, 1076-1084 (2006).
12. Haynesworth, S.E., Baber, M.A. & Caplan, A.I. Cytokine expression by human marrow-derived mesenchymal progenitor cells in vitro: effects of dexamethasone and IL-1 alpha. J Cell Physiol 166, 585-592 (1996).
13. Yeo, R.W., Lai, R.C., Tan, K.H. & Lim, S.K. Exosome: a novel and safer therapeutic refinement of mesenchymal stem cell. Exosomes Microvesicles 1, 1-12 (2013).
14. Bruno, S., et al. Mesenchymal stem cell-derived microvesicles protect against acute tubular injury. J Am Soc Nephrol 20, 1053-1067 (2009).
15. Akyurekli, C., et al. A Systematic Review of Preclinical Studies on the Therapeutic Potential of Mesenchymal Stromal Cell-Derived Microvesicles. Stem Cell Rev and Rep 11, 150-160 (2015).
16. Wubbolts, R., et al. Proteomic and biochemical analyses of human B cell-derived exosomes. Potential implications for their function and multivesicular body formation. J Biol Chem 278, 10963-10972 (2003).
17. de Gassart, A., Geminard, C., Fevrier, B., Raposo, G. & Vidal, M. Lipid raft-associated protein sorting in exosomes. Blood 102, 4336-4344 (2003).
18. Simons, M. & Raposo, G. Exosomes--vesicular carriers for intercellular communication. Curr Opin Cell Biol 21, 575-581 (2009).
19. Lopatina, T., et al. Platelet-derived growth factor regulates the secretion of extracellular vesicles by adipose mesenchymal stem cells and enhances their angiogenic potential. Cell Commun Signal 12, 26 (2014).
20. Ohtake, S., Kita, Y. & Arakawa, T. Interactions of formulation excipients with proteins in solution and in the dried state. Advanced Drug Delivery Reviews 63, 1053-1073 (2011).
21. Lai, R., et al. Mesenchymal Stem Cell Exosomes: The Future MSC-Based Therapy? in Mesenchymal Stem Cell Therapy (ed. Chase, L.G.V., Mohan C. ) 39-62 (Humana Press, 2013).
22. Sahoo, S., et al. Exosomes from human CD34(+) stem cells mediate their proangiogenic paracrine activity. Circ Res 109, 724-728 (2011).
23. Gaber, M.H. Effect of γ-irradiation on the molecular properties of bovine serum albumin. Journal of Bioscience and Bioengineering 100, 203-206 (2005).
24. Lai, R.C., et al. MSC secretes at least 3 EV types each with a unique permutation of membrane lipid, protein and RNA. 2016 (2016).

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

## Claims

1. A method for lyophilising an exosome, the method comprising the steps of:
(a) providing an exosome suspension in a lyophilisation buffer comprising trehalose at a concentration of 4% w/v;
(b) freezing the exosome suspension; and
(c) removing water from the frozen suspension by freeze drying;
in which the lyophilised exosome exhibits at least one biological activity of an exosome.

2. The method according to Claim 1, in which:
(i) the exosome exhibits 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 95% or more of the biological activity when reconstituted;
(ii) the exosome exhibits the biological activity, such as therapeutic activity, when stored at a temperature of 0°C or higher, 4°C or higher, 10°C or higher, 15°C or higher, 20°C or higher, 25°C or higher, 30°C or higher, 35°C or higher, 40°C or higher; or
(iii) the exosome exhibits a biological activity when stored for 1 day or more, 5 days or more, 7 days or more, 10 days or more, 14 days or more, 20 days or more, 21 days or more, 25 days or more, 28 days or more, 30 days or more, 35 days or more, or 40 days or more;
as compared to an exosome which has not been lyophilised.

3. The method according to any preceding claim, in which the biological activity comprises:
(a) CD73 enzymatic activity and in which the exosome exhibits 20% or more, such as 25% or more, 30% or more, 35% or more, 40% or more, 45% or more, 50% or more, 55% or more or 60% or more of CD73 enzymatic activity after 55 days of storage at 40°C, preferably when compared to the activity after 1 day of storage at 40°C;
(b) induction of polarization of CD4+ T cells to regulatory T cells (Tregs), in which the exosome exhibits 20% or more, such as 25% or more, 30% or more, 35% or more, 40% or more, 45% or more, 50% or more, 55% or more or 60% or more of activity to induce polarization of CD4+ T cells to regulatory T cells (Tregs) after 3 weeks of storage at 40°C, preferably when compared with a non-lyophilized exosome stored at - 80°C; or
(c) cardioprotection, in which the exosome exhibits 20% or more, such as 25% or more, 30% or more, 35% or more, 40% or more, 45% or more, 50% or more, 55% or more or 60% or more of cardioprotective activity, preferably when compared with a non-lyophilized exosome stored at -80°C, preferably in which the cardioprotection is assayed using an *ex vivo* Landerdorf model of ischemia-reperfusion injury, preferably by the degree of hypercontracture over post-reperfusion time.

4. The method according to any preceding claim, in which the exosome comprises a mesenchymal stem cell (MSC) exosome.

5. The method according to any preceding claim, in which:
(i) step (b) comprises freezing the exosome suspension at a temperature of -10°C or lower, -15°C or lower or -20°C or lower;
(ii) step (b) comprises freezing the exosome suspension for 1 hour or more, 2 hours or more or 3 hours or more;
(iii) step (c) comprises freeze drying at a temperature of -10°C or lower, -15°C or lower, -20°C or lower, -25°C or lower, -30°C or lower, -35°C or lower, -40°C or lower or -45°C or lower;
(iv) step (c) comprises freeze drying at a pressure of 1 mbar or lower, 0.5 mbar or lower, 0.25 mbar or lower, 0.1 mbar or lower or 0.05 mbar or lower;
(v) step (c) comprises freeze drying at a temperature of -10°C or lower, -15°C or lower, -20°C or lower, -25°C or lower, -30°C or lower, -35°C or lower, -40°C or lower or -45°C or lower; or
(vi) step (c) comprises freeze drying for 5 hours or longer, 10 hours or longer, 15 hours or longer or 20 hours or longer.

6. The method according to any preceding claim, in which step (b) comprises freezing the exosome suspension at about -20°C for about 3 hours and/or step (c) comprises freeze drying at < 0.05mbar for about 12 to 24 hours

7. The method according to any preceding claim, in which the method further comprises a step before step (c) of transferring the frozen suspension to a freeze dryer pre-cooled to -48°C.

8. The method according to any preceding claim, in which the method further comprises:
(a) storing the lyophilised exosome at a temperature of 4°C or higher, such as 10°C or higher, 15°C or higher, 20°C or higher, 25°C or higher, 30°C or higher, 35°C or higher or 40°C or higher;
(b) storing the lyophilised exosome in substantially dry conditions, such as in a desiccator, preferably in which the substantially dry conditions comprise storage in air with a relative humidity of 5% or less, such as 4% or less, 3% or less, 2% or less or 1% or less; or
(c) storing the lyophilised exosome in an inert gas, such as nitrogen.

9. The method according to any preceding claim, in which the lyophilisation buffer further comprises one or more of a buffer, for example a phosphate buffer, such as at pH 6.5-7.5, an amino acid for example L-glutamic acid, such as at 0.0049M and a protein binding stabiliser, for example human serum albumin such as at 1% w/v.

10. The method according to any preceding claim, in which the method further comprises a step of viral clearance, such as by exposure to gamma radiation, X-ray irradiation or ethylene oxide.

11. The method according to any preceding claim, comprising a step before step (b) of coating an object with the exosome suspension, such that removal of water from the frozen suspension deposits the exosome on the object, preferably in which the object comprises a scaffold made of a biocompatible material.

12. The method according to Claim 11, in which the object is selected from the group consisting of: a collagen membrane, a surgical dressing, a prosthetic bone replacement device, a cardiovascular device, a stent and a balloon catheter.

## Patentansprüche

1. Verfahren zum Lyophilisieren eines Exosoms, wobei das Verfahren die Schritte umfasst:
(a) Bereitstellen einer Exosomensuspension in einem Lyophilisationspuffer, der Trehalose in einer Konzentration von 4 % w/v umfasst;
(b) Einfrieren der Exosomensuspension; und
(c) Entfernen des Wassers aus der gefrorenen Suspension durch Gefriertrocknung;
wobei das lyophilisierte Exosom mindestens eine biologische Aktivität eines Exosoms aufweist.

2. Verfahren nach Anspruch 1, bei dem das Exosom im Vergleich zu einem nicht lyophilisierten Exosom:
(i) im rekonstituierten Zustand 5 % oder mehr, 10 % oder mehr, 15 % oder mehr, 20 % oder mehr, 25 % oder mehr, 30 % oder mehr, 35 % oder mehr, 40 % oder mehr, 45 % oder mehr, 50 % oder mehr, 55 % oder mehr, 60 % oder mehr, 65 % oder mehr, 70 % oder mehr, 75 % oder mehr, 80 % oder mehr, 85 % oder mehr, 90 % oder mehr oder 95 % oder mehr der biologischen Aktivität aufweist;
(ii) die biologische Aktivität, wie etwa therapeutische Aktivität, aufweist, wenn es bei einer Temperatur von 0°C oder höher, 4°C oder höher, 10°C oder höher, 15°C oder höher, 20°C oder höher, 25°C oder höher, 30°C oder höher, 35°C oder höher, 40°C oder höher gelagert wird; oder
(iii) eine biologische Aktivität aufweist, wenn es für 1 Tag oder länger, 5 Tage oder länger, 7 Tage oder länger, 10 Tage oder länger, 14 Tage oder länger, 20 Tage oder länger, 21 Tage oder länger, 25 Tage oder länger, 28 Tage oder länger, 30 Tage oder länger, 35 Tage oder länger oder 40 Tage oder länger gelagert wird.

3. Verfahren nach einem der vorhergehenden Ansprüche, bei dem die biologische Aktivität umfasst:
(a) CD73-Enzymaktivität, wobei das Exosom 20 % oder mehr, wie etwa 25 % oder mehr, 30 % oder mehr, 35 % oder mehr, 40 % oder mehr, 45 % oder mehr, 50 % oder mehr, 55 % oder mehr oder 60 % oder mehr CD73-Enzymaktivität nach 55 Tagen Lagerung bei 40 °C aufweist, vorzugsweise im Vergleich zur Aktivität nach 1 Tag Lagerung bei 40 °C;
(b) Induktion der Polarisierung von CD4+-T-Zellen zu regulatorischen T-Zellen (Tregs), wobei das Exosom 20 % oder mehr, wie etwa 25 % oder mehr, 30 % oder mehr, 35 % oder mehr, 40 % oder mehr, 45 % oder mehr, 50 % oder mehr, 55 % oder mehr oder 60 % oder mehr der Aktivität zur Induktion der Polarisierung von CD4+-T-Zellen zu regulatorischen T-Zellen (Tregs) nach 3 Wochen Lagerung bei 40 °C aufweist, vorzugsweise im Vergleich zu einem bei -80 °C gelagerten nicht lyophilisierten Exosom; oder
(c) Kardioprotektion, bei der das Exosom eine kardioprotektive Aktivität von 20 % oder mehr, wie etwa 25 % oder mehr, 30 % oder mehr, 35 % oder mehr, 40 % oder mehr, 45 % oder mehr, 50 % oder mehr, 55 % oder mehr oder 60 % oder mehr aufweist, vorzugsweise im Vergleich zu einem nicht lyophilisierten, bei -80°C gelagerten Exosom, wobei die kardioprotektive Wirkung vorzugsweise unter Verwendung eines Ex-vivo-Landerdorf-Modells der Ischämie-Reperfusionsschädigung, vorzugsweise durch den Grad der Hyperkontraktion über die Post-Reperfusionszeit, bestimmt wird.

4. Verfahren nach einem der vorhergehenden Ansprüche, bei dem das Exosom ein Exosom aus mesenchymalen Stammzellen (MSC) umfasst.

5. Verfahren nach einem der vorhergehenden Ansprüche, bei dem:
(i) Schritt (b) das Einfrieren der Exosomensuspension bei einer Temperatur von -10°C oder weniger, -15°C oder weniger oder -20°C oder weniger umfasst;
(ii) Schritt (b) das Einfrieren der Exosomensuspension für 1 Stunde oder länger, 2 Stunden oder länger oder 3 Stunden oder länger umfasst;
(iii) Schritt (c) das Gefriertrocknen bei einer Temperatur von -10°C oder niedriger, -15°C oder niedriger, -20°C oder niedriger, -25°C oder niedriger, -30°C oder niedriger, -35°C oder niedriger, -40°C oder niedriger oder -45°C oder niedriger umfasst;
(iv) Schritt (c) das Gefriertrocknen bei einem Druck von 1 mbar oder niedriger, 0,5 mbar oder niedriger, 0,25 mbar oder niedriger, 0,1 mbar oder niedriger oder 0,05 mbar oder niedriger umfasst;
(v) Schritt (c) das Gefriertrocknen bei einer Temperatur von -10°C oder niedriger, -15°C oder niedriger, -20°C oder niedriger, -25°C oder niedriger, -30°C oder niedriger, -35°C oder niedriger, -40°C oder niedriger oder -45°C oder niedriger umfasst; oder
(vi) Schritt (c) das Gefriertrocknen für 5 Stunden oder länger, 10 Stunden oder länger, 15 Stunden oder länger oder 20 Stunden oder länger umfasst.

6. Verfahren nach einem der vorhergehenden Ansprüche, bei dem Schritt (b) das Einfrieren der Exosomensuspension bei etwa -20°C für etwa 3 Stunden umfasst und/oder Schritt (c) das Gefriertrocknen bei < 0,05 mbar für etwa 12 bis 24 Stunden umfasst.

7. Verfahren nach einem der vorhergehenden Ansprüche, bei dem das Verfahren ferner einen Schritt vor Schritt (c) umfasst, bei dem die gefrorene Suspension in einen auf -48°C vorgekühlten Gefriertrockner überführt wird.

8. Verfahren nach einem der vorhergehenden Ansprüche, wobei das Verfahren ferner umfasst:
(a) Lagern des lyophilisierten Exosoms bei einer Temperatur von 4°C oder höher, wie etwa 10°C oder höher, 15°C oder höher, 20°C oder höher, 25°C oder höher, 30°C oder höher, 35°C oder höher oder 40°C oder höher;
(b) Lagern des lyophilisierten Exosoms unter im wesentlichen trockenen Bedingungen, wie etwa in einem Exsikkator, wobei die im wesentlichen trockenen Bedingungen die Lagerung in Luft mit einer relativen Luftfeuchtigkeit von 5 % oder weniger, wie 4 % oder weniger, 3 % oder weniger, 2 % oder weniger oder 1 % oder weniger umfassen; oder
(c) Lagern des lyophilisierten Exosoms in einem Inertgas, wie z. B. Stickstoff.

9. Verfahren nach einem der vorhergehenden Ansprüche, bei dem der Lyophilisationspuffer ferner einen oder mehrere Puffer, z.B. einen Phosphatpuffer, etwa mit einem pH-Wert von 6,5-7,5, eine Aminosäure, z.B. L-Glutaminsäure, z.B. mit einem Gehalt von 0,0049M, und einen Proteinbindungsstabilisator, z.B. Humanserumalbumin, z.B. mit einem Gehalt von 1 % w/v, umfasst.

10. Verfahren nach einem der vorhergehenden Ansprüche, wobei das Verfahren ferner einen Schritt des Virenabtötens umfasst, z.B. durch Gammastrahlen-, Röntgenstrahlen- oder Ethylenoxid-Exposition.

11. Verfahren nach einem der vorhergehenden Ansprüche, umfassend einen Schritt vor Schritt (b) des Beschichtens eines Objekts mit der Exosomensuspension, so dass der Entzug von Wasser aus der gefrorenen Suspension das Exosom auf dem Objekt ablagert, wobei das Objekt vorzugsweise ein Gerüst aus einem biokompatiblen Material umfasst.

12. Verfahren nach Anspruch 11, bei dem das Objekt ausgewählt ist aus der Gruppe bestehend aus: einer Kollagenmembran, einem chirurgischen Verband, einer prothetischen Knochenersatzvorrichtung, einer kardiovaskulären Vorrichtung, einem Stent und einem Ballonkatheter.

## Revendications

1. Procédé pour lyophiliser des exosomes, le procédé comprenant les étapes consistant à :
(a) fournir une suspension d'exosomes à un tampon de lyophilisation comprenant du tréhalose à une concentration de 4 % p/v ;
(b) congeler la suspension d'exosomes ;
(c) éliminer l'eau à partir de la suspension congelée par lyophilisation ; dans lequel les exosomes lyophilisés présentent au moins une activité biologique d'un exosome.

2. Procédé selon la revendication 1, dans lequel :
(i) les exosomes présentent 5 % ou plus, 10 % ou plus, 15 % ou plus, 20 % ou plus, 25 % ou plus, 30 % ou plus, 35 % ou plus, 40 % ou plus, 45 % ou plus, 50 % ou plus, 55 % ou plus, 60 % ou plus, 65 % ou plus, 70 % ou plus, 75 % ou plus, 80 % ou plus, 85 % ou plus, 90 % ou plus, ou 95 % ou plus d'activité biologique lorsqu'ils sont reconstitués ;
(ii) les exosomes présentent l'activité biologique, telle qu'une activité thérapeutique, lorsqu'ils sont stockés à une température égale ou supérieure à 0°C, égale ou supérieure à 4°C, égale ou supérieure à 10°C, égale ou supérieure à 15°C, égale ou supérieure à 20°C, égale ou supérieure à 25°C, égale ou supérieure à 30°C, égale ou supérieure à 35°C, égale ou supérieure à 40°C ; ou
(iii) les exosomes présentent une activité biologique lorsqu'ils sont stockés pendant 1 jour ou plus, 5 jours ou plus, 7 jours ou plus, 10 jours ou plus, 14 jours ou plus, 20 jours ou plus, 21 jours ou plus, 25 jours ou plus, 28 jours ou plus, 30 jours ou plus, 35 jours ou plus, ou 40 jours ou plus ;
par rapport à des exosomes qui n'ont pas été lyophilisés.

3. Procédé selon l'une quelconque des revendications précédentes, dans lequel l'activité biologique comprend :
(a) l'activité enzymatique de CD73, et les exosomes présentant 20 % ou plus, comme 25 % ou plus, 30 % ou plus, 35 % ou plus, 40 % ou plus, 45 % ou plus, 50 % ou plus, 55 % ou plus ou 60 % ou plus d'activité enzymatique de CD73 après 55 jours de stockage à 40°C, de préférence par comparaison à l'activité après 1 jour de stockage à 40°C ;
(b) une induction de polarisation de lymphocytes T CD4+ par rapport à des lymphocytes T régulateurs (Tregs), les exosomes présentant 20 % ou plus, comme 25 % ou plus, 30 % ou plus, 35 % ou plus, 40 % ou plus, 45 % ou plus, 50 % ou plus, 55 % ou plus ou 60 % ou plus d'activité pour induire une polarisation de lymphocytes T CD4+ par rapport à des lymphocytes T régulateurs (Tregs) après 3 semaines de stockage à 40°C, de préférence par comparaison avec des exosomes non lyophilisés stockés à -80°C ; ou
(c) une cardioprotection, les exosomes présentant 20 % ou plus, comme 25 % ou plus, 30 % ou plus, 35 % ou plus, 40 % ou plus, 45 % ou plus, 50 % ou plus, 55 % ou plus ou 60 % ou plus d'activité cardioprotectrice, de préférence par comparaison à des exosomes non lyophilisés stockés à -80°C, la cardioprotection étant de préférence dosée en utilisant un modèle de Landerdorf ex vivo de lésion ischémique-reperfusion, de préférence par le degré d'hypercontraction sur le temps de post-reperfusion.

4. Procédé selon l'une quelconque des revendications précédentes, dans lequel les exosomes comprennent des exosomes de cellules souches mésenchymateuses (MSC).

5. Procédé selon l'une quelconque des revendications précédentes, dans lequel :
(i) l'étape (b) comprend une congélation de la suspension d'exosomes à une température inférieure ou égale à -10°C, inférieure ou égale à -15°C ou inférieure ou égale à -20°C ;
(ii) l'étape b) consiste à congeler la suspension d'exosomes pendant 1 heure ou plus, 2 heures ou plus ou 3 heures ou plus ;
(iii) l'étape (c) comprend une lyophilisation à une température de -10°C ou moins, -15°C ou moins, -20°C ou moins, -25°C ou moins, -30°C ou moins, -35°C ou moins, -40°C ou moins ou -45°C ou moins ;
(iv) l'étape (c) comprend une lyophilisation à une pression de 1 mbar ou moins, 0,5 mbar ou moins, 0,25 mbar ou moins, 0,1 mbar ou moins ou 0,05 mbar ou moins;
(v) l'étape (c) comprend une lyophilisation à une température de -10°C ou moins, -15°C ou moins, -20°C ou moins, -25°C ou moins, -30°C ou moins, -35°C ou moins, -40°C ou inférieur ou -45°C ou inférieur ; ou
(vi) l'étape c) consiste à lyophiliser pendant 5 heures ou plus, 10 heures ou plus, 15 heures ou plus ou 20 heures ou plus.

6. Procédé selon l'une quelconque des revendications précédentes, dans lequel l'étape (b) comprend une congélation de la suspension d'exosomes à environ -20°C pendant environ 3 heures et/ou l'étape (c) comprend une lyophilisation à < 0,05 mbar pendant environ 12 à 24 heures.

7. Procédé selon l'une quelconque des revendications précédentes, dans lequel le procédé comprend en outre une étape préalable à l'étape (c) consistant à transférer la suspension congelée dans un lyophilisateur prérefroidi à -48°C.

8. Procédé selon l'une quelconque des revendications précédentes, dans lequel le procédé comprend en outre les étapes consistant à :
(a) stocker les exosomes lyophilisés à une température de 4°C ou plus, comme 10°C ou plus, 15°C ou plus, 20°C ou plus, 25°C ou plus, 30°C ou plus, 35°C ou plus ou 40°C ou plus ;
(b) stocker les exosomes lyophilisés dans des conditions sensiblement sèches, comme dans un dessiccateur, les conditions sensiblement sèches comprenant de préférence un stockage dans de l'air avec une humidité relative de 5 % ou moins, comme 4 % ou moins, 3 % ou moins, 2 % ou moins ou 1 % ou moins ; ou
(c) stocker les exosomes lyophilisés dans un gaz inerte, comme de l'azote.

9. Procédé selon l'une quelconque des revendications précédentes, dans lequel le tampon de lyophilisation comprend en outre un ou plusieurs parmi un tampon, par exemple un tampon de phosphate, comme à un pH de 6,5 à 7,5, un acide aminé, par exemple l'acide L-glutamique, comme à 0,0049 M, et un stabilisant de liaison aux protéines, par exemple l'albumine sérique humaine, comme à 1 % p/v.

10. Procédé selon l'une quelconque des revendications précédentes, dans lequel le procédé comprend en outre une étape de clairance virale, comme par une exposition à un rayonnement gamma, à une irradiation aux rayons X ou à de l'oxyde d'éthylène.

11. Procédé selon l'une quelconque des revendications précédentes, comprenant une étape avant l'étape (b) consistant à revêtir un objet avec la suspension d'exosomes, de telle sorte que l'élimination de l'eau à partir de la suspension congelée dépose les exosomes sur l'objet, de préférence dans lequel l'objet comprend un échafaudage constitué d'un matériau biocompatible.

12. Procédé selon la revendication 11, dans lequel l'objet est sélectionné dans le groupe constitué d'une membrane de collagène, d'un pansement chirurgical, d'un dispositif de remplacement osseux prothétique, d'un dispositif cardiovasculaire, d'une endoprothèse vasculaire et d'un cathéter à ballonnet.
